# EUROPEAN PATENT APPLICATION

(11) **EP 4 317 441 A1**
(43) Date of publication of application: **07.02.2024**
(21) Application number: 22776010.5
(22) Date of filing: 21.03.2022
(51) Int. Cl.: C12N 15/74, C07K 14/47, C07K 14/54, A61P 35/00, A61K 35/74, A61K 33/243

(54) **NOVEL RECOMBINANT STRAIN OF MYCOBACTERIUM SMEGMATIS AND USE OF SAME**

(30) Priority: 22.03.2021 KR 20210036586
(71) Applicant: Clipsbnc Co., Ltd, Seoul 04168 (KR)
(72) Inventor: KIM, Bum-Joon, Seoul 06001 (KR); AHN, Dong Ho, Seoul 04701 (KR); JEONG, Hyein, Seoul 03746 (KR); SEO, Hyejun, Seoul 02846 (KR); KIM, Jae Hee, Incheon 22370 (KR)
(74) Representative: V.O.
(86) International application number: PCT/KR2022/003908
(87) International publication number: WO 2022/203308

(57) **Abstract**

The present invention relates to a recombinant *Mycobacterium* strain co-expressing MIF and IL-7, and a composition for preventing or treating cancer containing the same as an active ingredient. The present invention induces a maximized anticancer immune response by stably expressing MIF and IL-7 through a mycobacteria-derived replicable plasmid, specifically, a pMyong2 shuttle vector developed by the present inventors. Accordingly, the present invention may be usefully used as an efficient anticancer live vaccine composition that induces multiple cellular and humoral immune responses through single administration of the recombinant strain.

## Description

### Technical Field

The present invention relates to a recombinant *Mycobacterium smegmatis* strain that induces a maximized immune response against cancer cells by co-expressing macrophage migration inhibitory factor (MIF) and interleukin-7 (IL-7).

### Background Art

According to Global Cancer Report 2018 released by the WHO, in 2018 alone, about 18 million new cancer patients occurred worldwide, and the total number of cancer patients reached about 44 million, of which 9.6 million patients died, indicating that cancer is the second leading cause of death from disease. Cancer is broadly divided into blood cancer, carcinomas that occur in epithelial cells, and sarcomas that are inflammatory tumors that occur in connective tissues such as bone, cartilage, lymph gland, muscle, and blood vessel, all of which are hyperproliferative diseases in which cancer cells have a fast growth rate and divide indefinitely. Although the normal human immune system is able to kill up to 10 million tumor cells generated in the body, there is a limit to completely eliminating cancer only by a normal immune response, due to the rapid proliferation of cancer cells.

Immunotherapy can be classified into an active immunotherapy in which a patient actively produces antibodies and sensitized lymphocytes, and a passive immunotherapy in which a patient accepts the immune response products already formed in another individual. It is also classified into specific therapy and non-specific therapy depending on whether the agent used has specificity for the therapeutic target. Among non-specific active immunotherapies, cytokine therapy aims to induce production and secretion of recombinant cytokines, thereby stimulating cytotoxic T-lymphocytes and activating major histocompatibility complex antigens.

Tumor immunotherapy began in 1891 when Coley discovered that bacterial infection of cancer patients induces temporary remission from cancer. In the 1960s, it was found that BCG eliminates some cancers. Non-tuberculous mycobacteria have excellent immune-inducing ability due to their complex cell wall components, and thus are able to efficiently activate macrophages or T cells to induce the secretion of various cytokines, thereby activating natural killer cells to kill cancer cells. BCG has been used until now after being approved for use against bladder cancer in the 1990s, and shows only limited effects on other tumors except for early bladder cancer.

Throughout the specification, a number of publications and patent documents are referred to and cited. The disclosure of the cited publications and patent documents is incorporated herein by reference in its entirety to more clearly describe the state of the related art and the present disclosure.

### DISCLOSURE

### Technical Problem

The present inventors have made extensive research efforts to develop an effective immunotherapeutic anticancer agent that induces multiple immune responses by single administration thereof. As a result, the present inventors have found that, when two cytokine genes, macrophage migration inhibitory factor (MIF), which regulates the innate cell-mediated immune response, and interleukin-7 (IL-7), which induces B cell and T cell differentiation, are inserted into a plasmid having a mycobacterial origin of replication and expressed in a recombinant *Mycobacterium* strain, the strain shows a maximized cancer cell killing effect by stimulating macrophages and inducing maturation of dendritic cells while significantly increasing the secretion of various inflammatory cytokines, thereby completing the present invention.

Therefore, an object of the present invention is to provide a recombinant *Mycobacterium* strain that co-expresses MIF and IL-7, and a composition for preventing or treating cancer containing the same as an active ingredient.

Other objects and advantages of the present invention will become more apparent from the following detailed description of the invention, the appended claims and the accompanying drawings.

### Technical Solution

According to one aspect of the present invention, the present invention provides a mycobacteria-derived replicable plasmid comprising: a nucleic acid molecule encoding macrophage migration inhibitory factor (MIF) or a functional portion thereof; a nucleic acid molecule encoding interleukin-7 (IL-7) or a functional portion thereof; or a combination thereof.

The present inventors have made extensive research efforts to develop an effective immunotherapeutic anticancer agent that induces multiple cellular and humoral immune responses by single administration thereof. As a result, the present inventors have found that, when two cytokine genes, macrophage migration inhibitory factor (MIF), which regulates the innate cell-mediated immune response, and interleukin-7 (IL-7), which induces B cell and T cell differentiation, are inserted into a plasmid having a mycobacterial origin of replication and expressed in a recombinant *Mycobacterium* strain, the strain shows a maximized cancer cell killing effect by stimulating macrophages and dendritic cells and significantly increasing the secretion of various inflammatory cytokines.

As used herein, the term "functional portion" is meant to encompass any partial fragment of any length that retains the biological activity of the MIF or IL-7 protein as a cytokine. Thus, the term "functional portion of the MIF or IL-7 protein" refers to a partial fragment of each protein that is capable of functioning as an inflammatory cytokine that regulates innate immunity by binding to CD74 or induces B cell and T cell differentiation.

As used herein, the term "protein" refers to a linear polymer of amino acid residues linked together by peptide bonds. The MIF and IL-7 proteins of the present invention may be wild-type human proteins whose amino acid sequences are published in public databases (e.g., National Center for Biotechnology Information), and are also construed to include amino acid sequences having substantial identity to the amino acid sequences of the proteins. Substantial identity refers to an amino acid sequence having at least 80%, specifically at least 80%, most specifically at least 95% homology, when aligning the known amino acid sequence with any other sequence to maximally correspond to each other and analyzing the aligned sequence using an algorithm commonly used in the art. In addition, protein variants having an amino acid sequence in which one or more amino acids are deleted, modified, substituted, or added may also be included as long as the amino acid sequence has the same or comparable biological activity as the above-described protein. Amino acid exchanges in proteins and peptides which do not generally alter the activity of such molecules are known in the art (H. Neurath, R. L. Hill, The Proteins, Academic Press, New York, 1979). The most commonly occurring amino acid exchanges are exchanges between Ala/Ser, Val/Ile, Asp/Glu, Thr/Ser, Ala/Gly, Ala/Thr, Ser/Asn, AlalVal, Ser/Gly, Tyr/Phe, Ala/Pro, Lys/Arg, Asp/Asn, Leu/Ile, Leu/Val, Ala/Glu, and Asp/Gly.

As used herein, the term "nucleic acid molecule" refers to a deoxyribonucleotide or ribonucleotide in single-strand or double-strand form, including analogs of natural nucleotides unless otherwise specified (Scheit, Nucleotide Analogs, John Wiley, New York(1980); Uhlman and Peyman, Chemical Reviews, 90:543-584(1990)). Each nucleic acid molecule encoding each of the MIF and IL-7 proteins of the present invention may be contained in each mycobacteria-derived replicable plasmid as a gene delivery system, or the two genes may be simultaneously inserted into one plasmid and expressed.

As used herein, the term "express" means allowing a subject to express an exogenous gene or artificially introducing an endogenous gene using a gene delivery system to increase the natural expression level of the endogenous gene, thereby making the gene replicable as an extrachromosomal factor or by chromosomal integration in the subject's cell. Accordingly, the term "expression" is synonymous with "transformation", "transfection" or "transduction".

As used herein, the term "gene delivery system" refers to a vehicle for introducing a desired target gene into a target cell to express the target gene. An ideal gene delivery system should be nontoxic to the human body, easily mass-produced, and efficiently deliver a gene.

As used herein, the term "gene delivery" means delivering the gene into cells, and has the same meaning as cellular transduction of the gene. At the tissue level, the term "gene delivery has the same meaning as spread of the gene. Thus, the gene delivery system of the present invention may be referred to as a gene transduction system and a gene spread system.

According to a specific embodiment of the present invention, the mycobacteria-derived plasmid that is used as a gene delivery system in the present invention further comprises an origin of replication comprising the nucleotide sequence of SEQ ID NO: 2 and the promoter of hsp65 or hsp60 gene.

As used herein, the term "origin of replication (ORI)" refers to a region of nucleotides necessary for replication of a plasmid, which is a specific conserved sequence in which replication within the genome starts. In the present invention, the origin of replication of a linear plasmid (pMyong2, GenBank accession number JQ657806) found in *M. yongonense* (DSM 45126T), a new bacterium of the genus *Mycobacterium,* is used (Lee H et al., PLoS One. 2015;10(3):e0122897).

As used herein, the term "promoter" refers to a DNA sequence that regulates the expression of a coding sequence or functional RNA. The promoter used in the plasmid of the present invention is the promoter of the heat shock protein 65 (hsp65) gene amplified from the genomic DNA of *M. bovis* BCG. In the shuttle vector of the present invention, the MIF and IL-7 protein-coding nucleotide sequences are operably linked to the promoter of the hsp65 gene. The term "operatively linked" refers to a functional linkage between a nucleic acid expression regulatory sequence such as a promoter and the nucleic acid sequence to be expressed, and through the linkage, the regulatory sequence regulates the transcription and/or translation of the other nucleic acid sequence.

The promoter of the hsp65 or hsp60 gene that is used in the present invention may be, for example, a *Mycobacterium*-derived promoter, more specifically a *M. tuberculosis*-derived promoter.

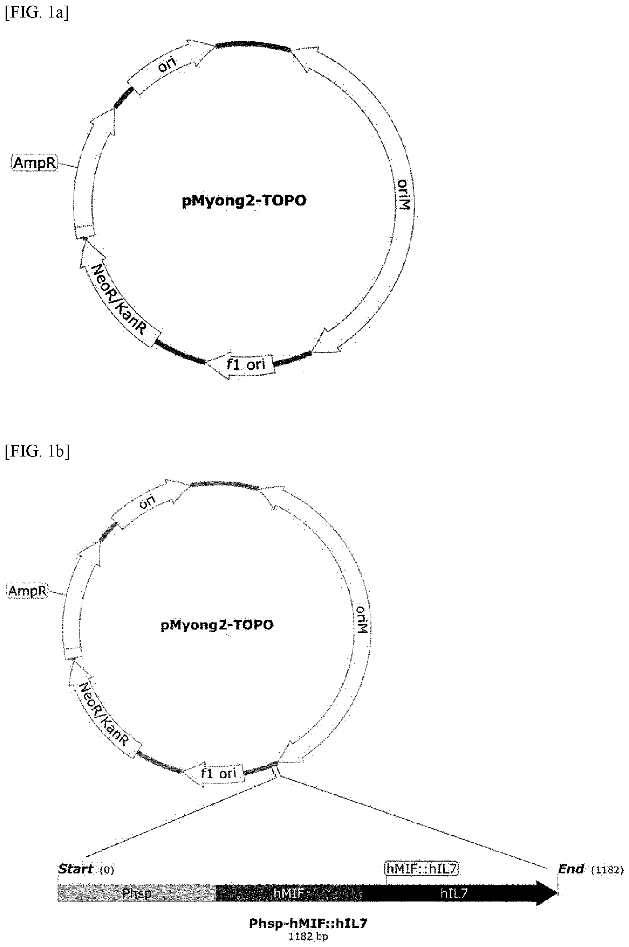

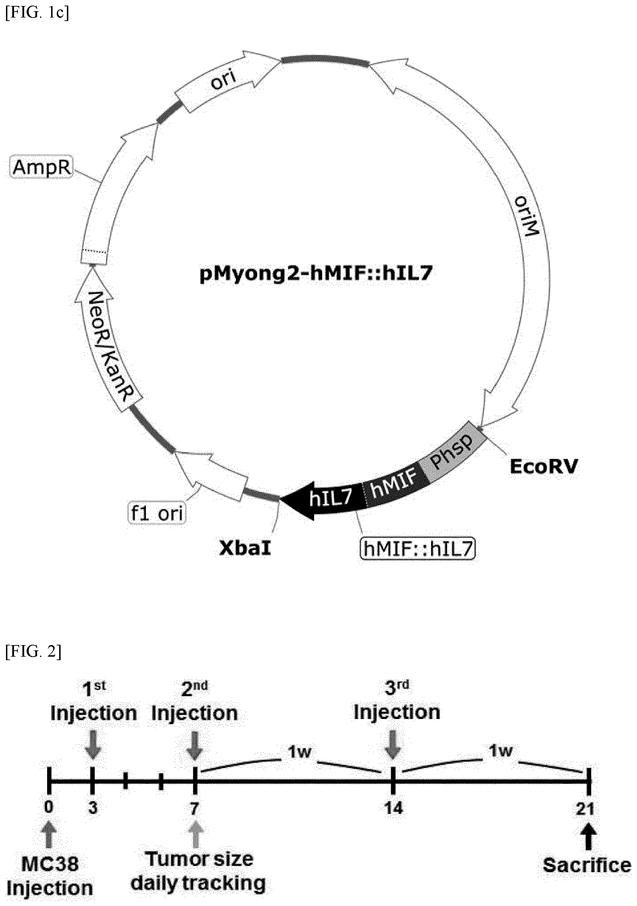

More specifically, the plasmid that is used in the present invention is a pMyong2 plasmid shown in FIG. 1a.

According to a specific embodiment of the present invention, the plasmid comprises the nucleotide sequence of SEQ ID NO: 1.

According to another aspect of the present invention, the present invention provides a recombinant *Mycobacterium* strain comprising the above-described plasmid of the present invention.

According to a specific embodiment of the present invention, the *Mycobacterium* is selected from the group consisting of *M. smegmatis*, *M. bovis* BCG, *M. avium*, *M. phlei, M. fortuitum*, *M. lufu*, *M. partuberculosis*, *M. habana*, *M. scrofulaceum,* and *M. intracellulare*. More specifically, the *Mycobacterium* is *M. smegmatis.*

According to still another aspect of the present invention, the present invention provides a composition for preventing or treating cancer containing the above-described *Mycobacterium* strain of the present invention as an active ingredient.

As used herein, the term "prevention" means inhibiting the occurrence of a disorder or disease in a subject who has never been diagnosed as having the disorder or disease, but is likely to suffer from such disorder or disease.

As used herein, the term "treatment" means (a) inhibiting the progress of a disorder, disease or symptom; (b) alleviating the disorder, disease or symptom; or (c) eliminating the disorder, disease or symptom. When the composition of the present invention is administered to a subject, it functions to inhibit the progress of cancer and the development of symptoms thereof by its maximized cancer cell killing effect through the induction of multiple immune responses, or to eliminate or alleviate them. Thus, the composition of the present invention may serve as a therapeutic composition for cancer alone, or may be administered in combination with other pharmacological ingredients and applied as a therapeutic aid for cancer. Accordingly, as herein used, the term "treatment" or "therapeutic agent" encompasses "treatment aid" or "therapeutic aid agent".

In addition, since the nucleic acid molecule encoding MIF in the composition of the present invention acts as an antigen to induce a humoral immune response that increases the antibody titer of anti-MIF IgG, the term "composition for preventing or treating cancer" as used herein has the same meaning as an "anticancer vaccine composition".

As used herein, the term "administration" or "administering" means administering a therapeutically effective amount of the composition of the present invention directly to a subject so that the same amount is formed in the subject's body.

As used herein, the term "therapeutically effective amount" refers to an amount of the composition containing a pharmacological ingredient sufficient to provide a therapeutic or prophylactic effect to a subj ect to whom the pharmaceutical composition of the present invention is to be administered. Accordingly, the term "therapeutically effective amount" is meant to encompass a "prophylactically effective amount".

As used herein, the term "subject" includes, without limitation, humans, mice, rats, guinea pigs, dogs, cats, horses, cows, pigs, monkeys, chimpanzees, baboons or rhesus monkeys. Specifically, the subject in the present invention is a human.

According to a specific embodiment of the present invention, the cancer that can be prevented or treated by the composition of the present invention is selected from the group consisting of breast cancer, liver cancer, lung cancer, colon cancer, bladder cancer, and pancreatic cancer.

According to a specific embodiment of the present invention, cancer that can be prevented or treated by the composition of the present invention may be metastatic cancer.

As used herein, the term "metastatic cancer" refers to a tumor newly formed while cancer cells break away from the primary tumor tissue, penetrate the surrounding blood vessels or lymphatic vessels, and spread to other parts of the body via these vessels. Since over 90% of deaths of cancer patients are due to metastasis from primary cancer, suppressing cancer metastasis to reduce the mortality of cancer patients is as important as treating primary cancer. As shown in the Examples described below, the composition of the present invention is able to effectively block the process of cancer metastasis by significantly reducing the migration and invasion ability of cancer cells and greatly reducing the expression of MMP-2 and MMP-9, which are factors related to cancer cell metastasis.

According to a specific embodiment of the present invention, the composition of the present invention further contains cisplatin or a pharmaceutically acceptable salt thereof.

According to the present invention, the recombinant *Mycobacterium* strain of the present invention, when co-administered with the low-molecular-weight anticancer drug cisplatin, exhibits a more efficient cancer cell killing ability by exhibiting a remarkable synergistic effect. The co-administration may be performed using a single formulation containing both the recombinant *Mycobacterium* strain of the present invention and cisplatin, or may be performed by administering separate formulations containing the recombinant *Mycobacterium* strain and cisplatin, respectively, simultaneously or sequentially with appropriate time delays in any order.

According to a specific embodiment of the present invention, the composition of the present invention further contains an immune checkpoint inhibitor.

As used herein, the term "immune checkpoint" refers to an intracellular signaling system that maintains self-tolerance and protects tissues from excessive immune responses that cause damage. Immune checkpoint proteins are cell membrane proteins that regulate immune checkpoint and are able to inhibit differentiation, proliferation, and activity of immune cells. Specifically, these immune checkpoint proteins are expressed in activated T cells and function to reduce T cell proliferation, cytokine secretion, and cytotoxicity, and inhibit excessive activity of T cells. Some immune checkpoints act as one of the main mechanisms by which tumor cells evade the patient's immune response. Accordingly, the term "immune checkpoint inhibitor" refers to an anticancer component or anticancer adjuvant component that blocks the expression or activity of an immune checkpoint protein, thereby enhancing T cell activity, thus promoting an antitumor immune response.

Examples of the immune checkpoint inhibitor that is used in the present invention include, but are not limited to, antibodies against cytotoxic T-lymphocyte antigen 4 (CTLA-4), programmed cell death 1 protein (PD-1), programmed cell death 1 ligand 1 (PD-L1), programmed cell death 1 ligand 2 (PD-L2), lymphocyte activation gene 3 (LAG3), B7-1, B7-H3, T cell immunoreceptor with Ig and ITIM domains (TIGIT), and T cell membrane protein 3 (TIM3) ).

More specifically, the immune checkpoint inhibitor is an anti-PD-L1 antibody. According to an exemplary embodiment of the present invention, as the anti-PD-L1 antibody, for example, anti-mouse PD-L1 B7-H1 (InvivoMab, catalog# BE0101) may be used.

According to yet another aspect of the present invention, the present invention provides a method for preventing or treating cancer comprising a step of administering the above-described composition of the present invention to a subject.

Since the recombinant *Mycobacterium* that is used in the present invention, the pharmaceutical composition containing the same, and the kind of cancer that can be prevented or treated thereby have already been described above, the description thereof will be omitted to avoid excessive redundancy.

### Advantageous Effects

The features and advantages of the present invention are summarized as follows:
(a) The present invention provides a recombinant *Mycobacterium* strain that co-expresses MIF and IL-7, and a composition for preventing or treating cancer containing the same as an active ingredient.
(b) The present invention induces a maximized anticancer immune response by stably expressing MIF and IL-7 through a mycobacteria-derived replicable plasmid, specifically, a pMyong2 shuttle vector developed by the present inventors.
(c) The present invention may be usefully used as an efficient anticancer live vaccine composition that induces multiple cellular and humoral immune responses through single administration of the recombinant strain.

### Brief Description of Drawings

FIG. 1 shows a schematic view of the map of a pMyong2 shuttle vector, which is a mycobacteria-derived replicable plasmid that is used in the present invention (FIG. 1a), the insertion position of Phsp-hMIF::hII,7 genes in a pMyong2-TOPO vector (FIG. 1b), and a schematic view of a pMyong2-hMIF::hIL7 plasmid into which these genes have been inserted (FIG. 1c).
FIG. 2 shows an animal experiment schedule for evaluating the vaccine effect of the recombinant strain of the present invention.
FIG. 3 illustrates a process of constructing a plasmid DNA expressing human MIF and human IL-7.
FIG. 4 shows the results of performing PCR analysis on *E. coli* colonies after cloning each of phsp-hMIF, phsp-IL-7, and phsp-TBMC proteins, and a fusion sequence of these proteins using pMV306 and pMyong2 vector.
FIG. 5 is a plasmid schematic diagram showing the results of analyzing the nucleotide sequence of extracted plasmid DNA by PCR after culturing *E. coli* colonies, in which a gene of a desired size was detected by colony PCR, in LB liquid medium.
FIG. 6 shows the results of PCR analysis performed to confirm gene expression in recombinant *M. smegmatis* expressing human MIF.
FIG. 7 shows the results of PCR analysis performed to confirm gene expression in recombinant *M. smegmatis* expressing human IL7.
FIG. 8 shows the results of comparing the hMIF and hIL7 expression levels of each recombinant *M. smegamtis* constructed in the present invention.
FIG. 9 shows the results of expressing various proteins in *M. bovis* BCG using recombinant pMV306 and pMyong2 vectors.
FIG. 10 shows the results of performing culture up to 10 passages with and without antibiotics in order to evaluate the stability of a protein expressed by *M smegmatis* pMyong2-hMIF::IL7.
FIG. 11 shows the results of evaluating the *in vivo* stability of a protein expressed by *M. smegmatis* pMyong2-hMIF::IL7.
FIG. 12 shows the results of measuring intracellular CFU (FIG. 12a) and evaluating the cell killing effect (FIG. 12b) after infecting macrophages with the recombinant strain of the present invention.
FIG. 13 shows the results of comparing the CFUs of *M smegmatis* wild-type and *M smegmatis* pMyong2-hMIF::IL7 in mice.
FIG. 14 shows the results of confirming the induction of maturation of dendritic cells by pMyong2 recombinant *M. smegmatis.*
FIG. 15 shows inflammatory cytokines secreted by pMyong2 recombinant *M. smegmatis.*
FIG. 16 shows the results of comparing the metabolic abilities of human-derived cancer cells (FIG. 16a) and mouse-derived cancer cells (FIG. 16b) infected with recombinant *M. smegmatis.*
FIG. 17 shows the cancer cell killing ability of the macrophages and dendritic cells stimulated with the recombinant strain of the present invention.
FIG. 18 shows the results of comparing the migration characteristics of various mouse-derived cancer cells (melanoma cells B16F10, breast cancer cells EO771, and colorectal cancer cells MC38).
FIG. 19 shows the decrease in invasion of MC38 cells by *M. smegmatis-*pMyong2_hMIF::IL7 infection.
FIG. 20 shows the anticancer effect of each recombinant *M. smegmatis* in a mouse tumor model.
FIG. 21 shows the results of evaluating the anticancer effect of *M. smegmatis-*pMyong2_hMIF::IL7 in an *in vivo* model.
FIG. 22 shows the results of evaluating the immune response (anti-human MIF IgG concentration) in serum of a mouse tumor model.
FIG. 23 shows the results of measuring cytokine secretion in serum of a mouse tumor model.
FIG. 24 shows mRNA expression of Cyclin D1, CD74, MMP-2, and MMP-9 in cancer tissue of a mouse tumor model.
FIG. 25 shows the results of measuring the expression of MIF and CD74 in cancer tissue of a mouse tumor model through immunohistochemical analysis.
FIG. 26 shows an increase in TNF-α-secreting CD8 T cells and NK cells in the spleen of a mouse tumor model.
FIG. 27 shows the results of measuring the proportions of immune cells in spleen (FIG. 27a) and tumor tissue (FIG. 27b).
FIG. 28 shows that CD4 T cells secreting IFN-γ and CD8 T cells secreting IFN-γ and TNF-α in tumor tissue are increased by administration of *M. smegmatis*_pMyong2-hMIF::II,7.
FIG. 29 shows the results of measuring the expression of granzyme B and Perforin-1 in tumor tissue.
FIG. 30 shows the anticancer effect of co-administration of the recombinant strain of the present invention and cisplatin, a commercially available anticancer drug.
FIG. 31 shows the results of measuring changes in body weight and spleen weight of mice upon co-administration of the recombinant strain of the present invention and cisplatin.
FIG. 32 shows changes in MIF concentration in serum upon co-administration of the recombinant strain of the present invention and cisplatin.
FIG. 33 shows the increase in anti-MIF antibody titer (total anti-MIF IgG) in serum upon co-administration of the recombinant strain of the present invention and cisplatin.
FIG. 34 shows an increase in the infiltration of Th1 helper T cells and cytotoxic T cells (FIGS. 34a and 34b) and an increase in the proportion of TCRγδ T cells (FIG. 34c), and indicates that the immune response in cancer tissues is increased upon co-administration of the recombinant strain of the present invention and cisplatin.

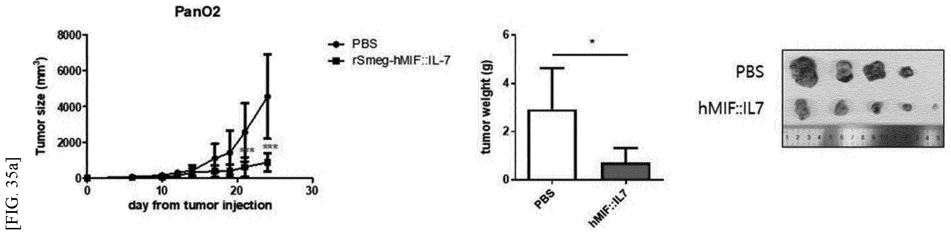

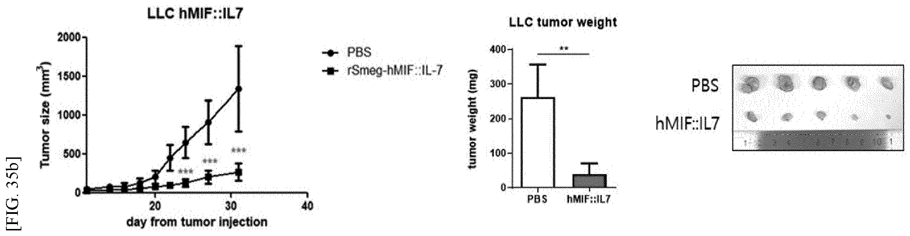

FIG. 35 shows the cancer growth inhibitory effect of *M. smegmatis-*pMyong2_hMIF::IL7 on PanO2 (FIG. 35a) and LLC (FIG. 35a).
FIG. 36 shows the immune response of the sera and splenocytes isolated from mouse models transplanted with PanO2 (FIG. 36a) and LLC (FIG. 36a).
FIG. 37 shows the results of measuring the proportion of IFNy-secreting TCRγδ T cells in tumor tissues of the PanO2-transplanted mouse model (FIG. 37a), the proportions of IFNy-secreting CD4 helper T cells and TNFα-secreting CD4 helper T cells in tumor and spleen tissues (FIGS. 37b and 37c), and the proportions of CD4 helper T cells and cytotoxic CD8 T cells secreting IFNy and TNFα in spleen tissue (FIG. 37d).
FIG. 38 shows the proportions of IFNy-secreting CD4 helper T cells, cytotoxic CD8 T cells, and TNFα-secreting CD4 helper T cells in the tumor tissue of an LLC-transplanted mouse model (FIG. 38a) and the proportions of CD4 helper T cells and cytotoxic CD8 T cells secreting IFNy and TNFα in the spleen tissue (FIGS. 38b and 38c).
FIG. 39 shows the MIF activity inhibitory effect of serum isolated from MC38-transplanted mice, and shows the result of measuring the biological activity of MIF in serum by tautomerase assay (FIG. 39a), the results of measuring the expression level of proteins related to proliferation and metastasis of intracellular MIF-related cancer cells (FIG. 39b), and the results of 7AAD/Annexin V apoptosis assay (FIG. 39c).

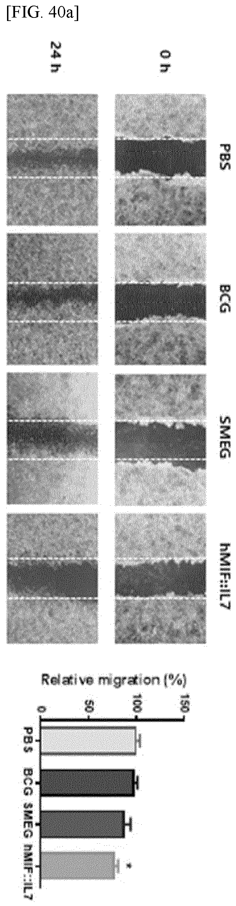

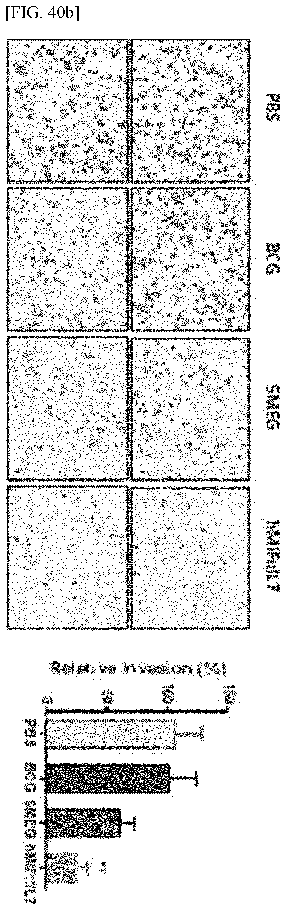

FIG. 40 shows the effects of serum isolated from MC38-transplanted mice on the inhibition of cancer cell migration (FIG. 40a) and invasion (FIG. 40b).
FIG. 41 shows protein and RNA expression in tumor tissue isolated from MC38-transplanted mice by Western blot measurement (FIG. 41a) and a heat map (FIG. 41b).
FIG. 42 shows cancer cell growth inhibitory efficacy (FIG. 42a), changes in humoral immune response and inflammatory cytokine concentration changes (FIG. 42b) and changes in serum MIF concentration (FIG. 42c) by co-administration of *M. smeginatis-*pMyong2_hMIF::IL7 and an anti-PD-L1 antibody, a commercially available anticancer drug.
FIG. 43 shows the results of observing the changes in immune profiles in tumor tissue by co-administration of *M. smegmatis*-pMyong2_hMIF::IL7 and an anti-PD-L1 antibody, a commercially available anticancer drug.
FIG. 44 shows the *in vitro* anticancer effect of the recombinant *M. smegmatis* strain of the present invention, and shows cytotoxicity against cancer cells (FIG. 44a), changes in cytokine expression in immune cells (FIG. 44b), and changes in MIF concentration (FIG. 44c).
FIG. 45 shows the results of comparing the metabolic abilities of various cancer cells infected with the recombinant *M. smegmatis* strain of the present invention.

### Mode for Invention

Hereinafter, the present invention will be described in more detail with reference to examples. These examples are only for explaining the present invention in more detail, and it will be apparent to those skilled in the art that the scope of the present invention according to the subject matter of the present invention is not limited by these examples.

### Example 1

### Experimental Methods

### Construction of recombinant M. smegmatis expressing human MIF or IL-7

### Construction of vectors expressing hMIF and IL-7

To construct a mycobacteria-shuttle vector expressing human macrophage migration inhibitory factor (MIF) and IL-7, the promoter of the heat shock protein (hsp) 65 gene was amplified from the genomic DNA of *M. bovis* BCG and ligated with the hMIF and IL-7 amplification products via overlapping PCR, thereby constructing phsp-hMIF and phsp-IL7 sequences. The constructed phsp-hMIF and phsp-IL-7 sequences were cloned into pMV306 and pMyong2_TOPO vectors, thereby constructing *mycobacteria-E. coli* shuttle vectors capable of expressing human MIF and/or IL-7 (pMV306-hMIF, pMyong2-hMIF, pMV306-IL7, pMyong2-IL7, pMV306-hMIF::hIL7, and pMyong2-hMIF::hIL7).

### Transformation of constructed vectors into various NTMs

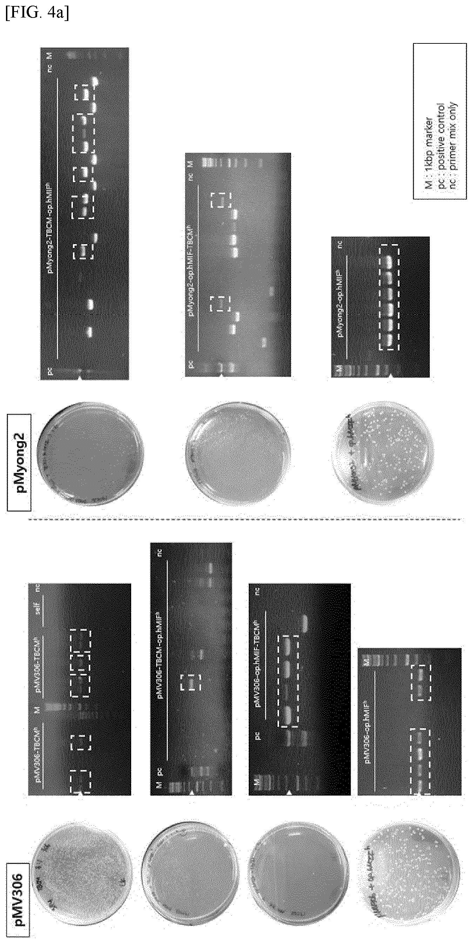

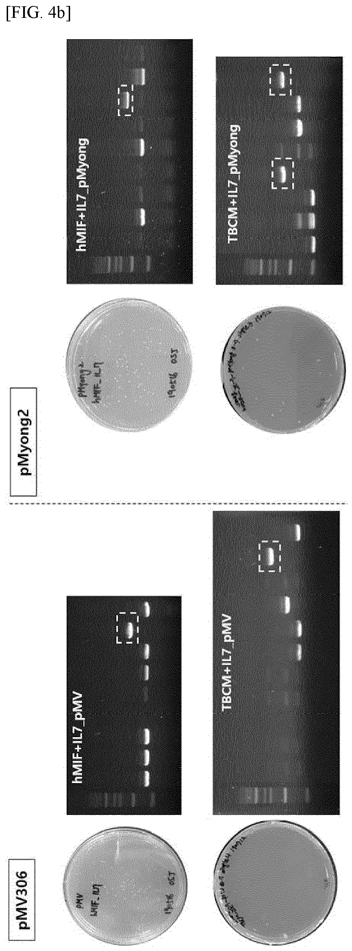

Each vector was transformed into *M. smegmatis* and *M. bovis* BCG by electroporation (Gene Pulser II; Bio-RAD, Hercules, CA, USA) to obtain transformed strains (FIG. 4). Each of the recombinant strain was passaged and maintained in kanamycin-selective 7H10 medium.

### ELISA analysis of expression of hMIF and IL-7 in recombinant strain

Each recombinant strain was lysed in B-PER buffer (Thermo Scientific, Rockford, IL, USA) supplemented with 100 µg/ml lysozyme, 5 U/ml DNase and proteinase inhibitors and was sonicated on ice (5 min, pulse: 0.3 sec, stop: 0.7 sec). The lysates were centrifuged at 13,000 rpm at 4°C for 15 minutes, and the supernatants were taken. The expression levels of hMIF and IL-7 were measured by ELISA using proteins extracted from each recombinant strain.

### Evaluation of stability of recombinant strains

### ELISA analysis of expression of hMIF and IL-7 in recombinant strain after cell infection

Mouse macrophages (J774A. 1) were seeded in a 6-well plate, and after 24 hours, the cells were infected with 10 M.O.I. (multiplicity of infection) of each recombinant strain. After 2 or 4 hours, the infected cells were washed with PBS, the extracellular bacteria were removed, and then the medium was replaced with a fresh medium. After 24 hours of infection, proteins were extracted from the cells, and the expression levels of hMIF and IL-7 by each recombinant strain were comparatively measured using hMIF and IL-7 ELISA kits.

### Evaluation of in vivo stability of recombinant strain

Mice were infected with each recombinant strain (5 x 10⁶) by intravenous (I.V.) route. After 1 week, the mice were sacrificed, and the spleen was harvested and homogenized. The cell solution at an appropriate dilution factor was plated on a solid medium, and then cultured in an incubator at 37°C to obtain a number of single colonies. For each colony, the gene sequence inserted into the plasmid vector was amplified by PCR using amplifiable primers, and then subjected to electrophoresis, and the probability of maintaining properties as a recombinant strain was calculated.

### Evaluation of safety of recombinant strains

### LDH assay and 7AAD staining

Mouse-derived macrophages (J774A.1) were seeded in a 6-well plate, and after 24 hours, the cells were infected with 10 M.O.I. of each recombinant strain. After 2 or 4 hours, the infected cells were washed with PBS, the extracellular bacteria were removed, and the medium was replaced with a fresh medium. At each time point, LDH assay was performed using the culture, the infected cells were stained with 7AAD and Annexin V, and cytotoxicity was evaluated by flow cytometry.

### Safety evaluation in vitro

A mouse-derived macrophage cell line (J774A.1) was seeded in a 6-well plate, and after 24 hours, the cell line was infected with 10 M.O.I. of each recombinant strain. After 4 hours, the infected cells were washed with PBS, extracellular bacteria were removed, and the medium was replaced with a fresh medium. At each time point, the cells were detached with 0.5% Triton X-100 in PBS, and the cell solution at an appropriate dilution factor was plated on 7H10 solid medium supplemented with OADC, and cultured in an incubator at 37°C for about 4 weeks, and colony forming unit (CFU) was comparatively measured through colony counting.

### Safety evaluation in vitro

Mice were infected intravenously with each recombinant strain (5 × 10⁶). After 1 week, the mice were sacrificed, the spleen was harvested and homogenized, and the cell solution at an appropriate dilution factor was plated on a solid medium and cultured at 37°C. Thereafter, the number of colonies was counted and CFU was comparatively measured.

### Evaluation of immune activity of recombinant strains

### Dendritic cell differentiation

Mouse femur and tibia were isolated, and bone marrow cells were isolated therefrom and cultured in IMDM medium supplemented with IL-4 and GM-CSF for 6 days to induce differentiation into dendrite cells. The CD11c marker in the differentiated dendritic cells was checked, and only those cells that were over 80% differentiated were used in the experiment.

### Evaluation of degree of maturation of dendritic cells upon infection with recombinant strain by flow cytometry

Differentiated dendritic cells were infected with each recombinant strain for 24 hours. The infected cells were detached, blocked for 30 minutes to inhibit non-specific antibody binding, and then stained with fluorescent antibodies against CD40, CD80, CD86 and type II MHC molecules for 30 minutes. Next, the cells were washed and suspended in FACS buffer, and then the intensity of each fluorescence was comparatively measured using the BD LSRFortessa instrument.

### Measurement of cytokines secreted by dendritic cells

ELISA was performed to measure secreted cytokines in a culture of dendritic cells infected with each recombinant strain. An ELISA 96-well plate was coated with a capture antibody for 24 hours, washed, and then blocked for about 1 hour by adding 1% BSA in PBS to each well. After washing, a culture of infected dendritic cells was added and cultured at room temperature for 2 hours. After washing again, the cells were incubated with a detection antibody at room temperature for 2 hours, and then incubated with horseradish-peroxidase (HRP)-conjugated streptavidin for 30 minutes to develop color. Then, absorbance was measured at 450 nm (TNF-α, IL-6, IL-10, IL-12, etc.).

### Observation of anticancer effects against various cancer cell lines

### Observation of direct cytotoxicity of recombinant strains to various cancer cell lines

In order to examine whether each recombinant strain directly increases cytotoxicity against cancer cells, each of human-derived breast cancer cell line MCF-7, liver cancer cell line HepG2, lung cancer cell line A549, mouse-derived colon cancer cell line MC38, bladder cancer cell line Mbt-2 was seeded in a 24-well plate, and after 24 hours, each cell line was infected with 1, 10, or 20 M.O.I. of each recombinant strain. After 4 hours, the infected cells were washed with PBS, extracellular bacteria were removed, and the medium was replaced with a fresh medium. At each time point, LDH assay was performed using the culture, the infected cells were stained with 7AAD and Annexin V, and cytotoxicity was evaluated by flow cytometry.

### Evaluation of cytotoxicity against cancer cell lines through macrophages

In order to examine whether the recombinant strain induces an immune response against cancer cells by stimulating macrophages, mouse macrophage J774A.1 was infected with each recombinant strain at 1, 10, or 20 M.O.I. After 4 hours, the infected cells were washed with PBS, extracellular bacteria were removed, and the medium was replaced with a new medium. Then, the infected macrophages were co-cultured with mouse-derived colorectal cancer cell line MC38 and bladder cancer cell line Mbt-2 for 24 hours,, and cytotoxicity was evaluated by measuring LDH in the cell culture.

### Observation of in vivo anticancer effect using MC38 mouse colorectal cancer cell line

1 × 10⁶ MC38 cells were injected subcutaneously (S.C.), and after 3 days, 7 days, and 14 days, 1 × 10⁷ CFU of each recombinant strain was subcutaneously inoculated a total of three times. The tumor size was measured until the end of the experiment, one week after the last injection of the recombinant strain, and the mice of each group were sacrificed and a tumor was harvested. At the end of the experiment, the anticancer effect of the recombinant strain was evaluated by comparing the tumor weight and the tumor size measured during the experiment (FIG. 2).

### Immune response in mice with induced cancer

### Measurement of cytokine expression

Mouse splenocytes in which the anticancer effect of each recombinant strain was observed were seeded in a 96-well plate and re-stimulated by treatment with MC38 cytolytic antigen at a concentration of 5 µg/ml. Cultures of the cells were collected at 24 or 72 hours and stored at -70°C. Thereafter, ELISA was performed for cytokines related to immune response activation (TNF-α, IFN-γ) or immunosuppression (IL-4, IL-10, etc.), and the expression patterns of these cytokines were comparatively analyzed.

### Observation of NK and T cells expressing cytokines

Mouse splenocytes in which the anticancer effect of each recombinant strain was observed were seeded in a 96-well plate, and re-stimulated with MC38 cytolytic antigen at a concentration of 5 µg/ml. After 48 hours, the cells were treated with brefeldin A, which traps proteins in the endoplasmic reticulum (ER), for 4 hours, and then stained with fluorescent antibodies against CD3, CD4, and CD8 molecules. Then, in order to stain intracellular IFN-y, the cells were fixed, permeabilized, and then stained with a fluorescent antibody against IFN-γ, and the expression level of IFN-γ was comparatively analyzed using BD LSRFortessa.

### Observation of effect of co-administration with anticancer drug

In order to maximize the anticancer effect of the recombinant strain, combination therapy with an anticancer drug was performed. 1 × 10⁶ MC38 cells were injected subcutaneously, and after 3 days, 7 days, and 14 days, 1 × 10⁷ CFU of the recombinant strain was subcutaneously inoculated a total of three times. To confirm the effect of co-administration, recombinant *M. smegmatis* and cisplatin (50 µg/kg) were intraperitoneally administered. The tumor size was measured until the end of the experiment, one week after the last injection of the recombinant strain, and the mice in each group were sacrificed and the tumor was harvested. At the end of the experiment, the anticancer effect of the recombinant strain was evaluated by comparing the tumor weight and the tumor size measured during the experiment.

### Experimental Results

### Generation of plasmid DNA expressing human MIF or human IL-7

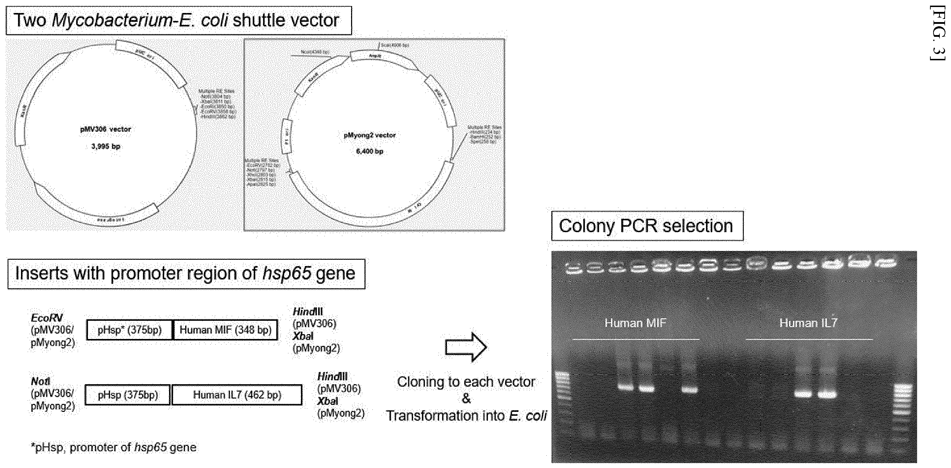

To construct recombinant *M. smegmatis* strains, plasmids containing human MIF or human IL-7 gene in the integration vector pMV306 and the novel mycobacteria-*E*. *coli* shuttle vector pMyong2 were constructed. To construct a mycobacteria-shuttle vector expressing human MIF and IL-7, the phsp-hMIF and phsp-IL7 sequences were constructed by amplifying the promoter of the hsp65 gene from the genomic DNA of *M. bovis* BCG (FIG. 3). In addition, TBCM, a protein derived from *Mycobacterium tuberculosis,* which can induce an immune response, was also constructed, and a vector expressing a fusion protein of hMIF, hIL7 and TBCM was also constructed.

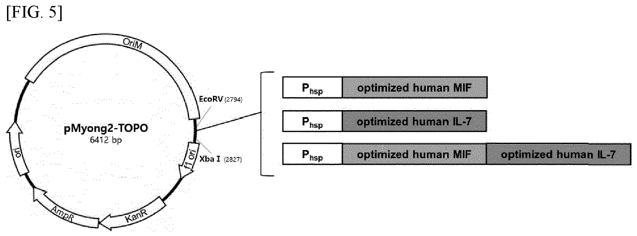

Mycobacteria-*E*. *coli* shuttle vectors capable of expressing human MIF and IL-7 were constructed by cloning the constructed phsp-hMIF, phsp-IL-7 and phsp-TBMC proteins and the fusion sequence of the three proteins into pMV306 and pMyong2 vectors. The constructed vectors were injected into *E. coli* by heat-shock, and then plated on LB solid medium containing kanamycin antibiotic. Colonies surviving in the kanamycincontaining medium including antibiotic resistance genes were selected, and PCR was performed with primers targeting the injected gene. *E. coli* colonies in which a gene of a desired size was detected by colony PCR were cultured in LB liquid medium, and then plasmid DNA was extracted and sequenced (FIG. 5). Through the above-described method, the following 12 types of plasmid DNA containing the nucleotide sequence of a protein-coding gene capable of enhancing an immune response were obtained: pMV306-hMIF, pMV306-hIL7, pMV306-TBCM, pMV306-hMIF::hIL7, pMV306-hMIF::TBCM, pMV306-TBCM::hMIF, pMyong2-hMIF, pMyong2-hIL7, pMyong2-TBCM, pMyong2-hMIF::hIL7, pMyong2-hMIF::TBCM, and pMyong2-TBCM::hMIF.

### Construction of recombinant M. smegmatis bacteria expressing human MIF or human IL-7

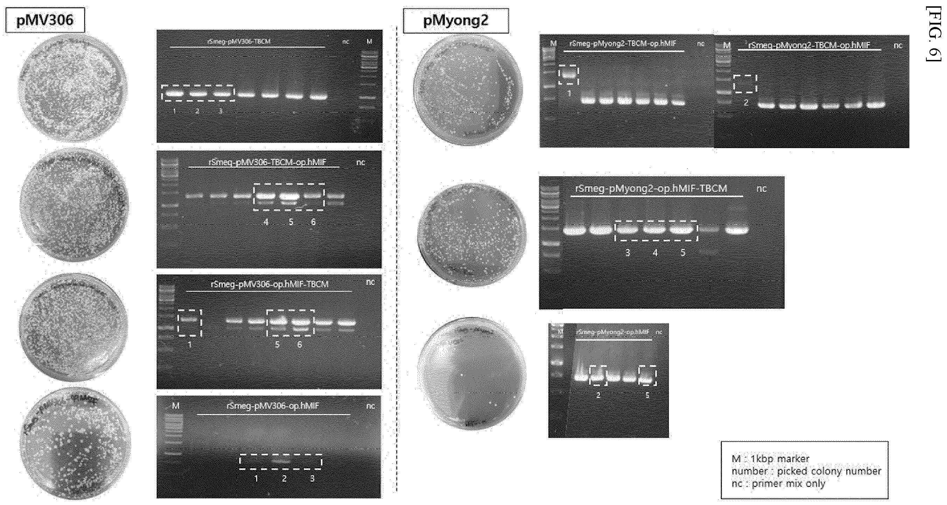

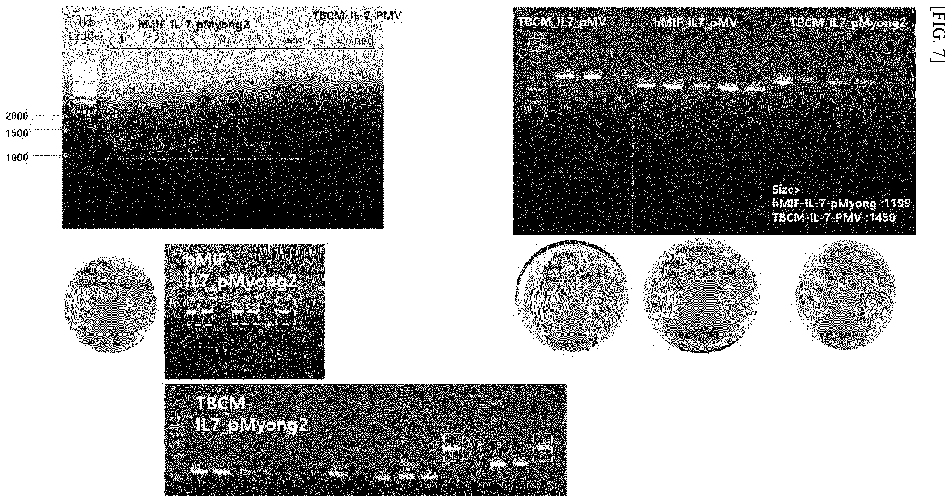

In order to generate recombinant *M. smegmatis* that induces an immune response, each vector whose nucleotide sequence was confirmed was transformed into *M. smegmatis* bacteria, and recombinant *M. smegmatis* bacteria expressing hMIF, TBCM, hMIF::TBCM, TBCM::hMIF (FIG. 6) and hIL7, hMIF::hIL7, TBCM: :hIL7 (FIG. 7) were selected on 7H10 solid medium containing kanamycin antibiotic. Whether the selected bacteria expressed the desired gene was analyzed by colony PCR, thus obtaining the following recombinant M. smegmatis strains expressing hMIF, hIL7, TBCM, and a fusion protein thereof: rSmeg-pMV306-hMIF, rSmeg-pMV306-hIL7, rSmeg-pMV306-TBCM, rSmeg-pMV306-hMIF::hIL7, rSmeg-pMV3 06-hMIF: : TBCM, rSmeg-pMV306-TBCM::hMIF, rSmeg-pMyong2-hMIF, rSmeg-pMyong2-hIL7, rSmeg-pMyong2-TBCM, rSmeg-pMyong2-hMIF::hIL7, rSmeg-pMyong2-hMIF::TBCM, and rSmeg-pMyong2-TBCM::hMIF.

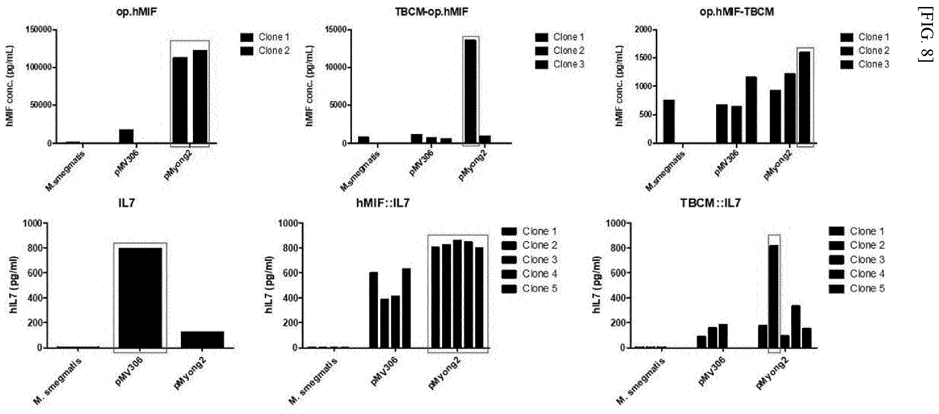

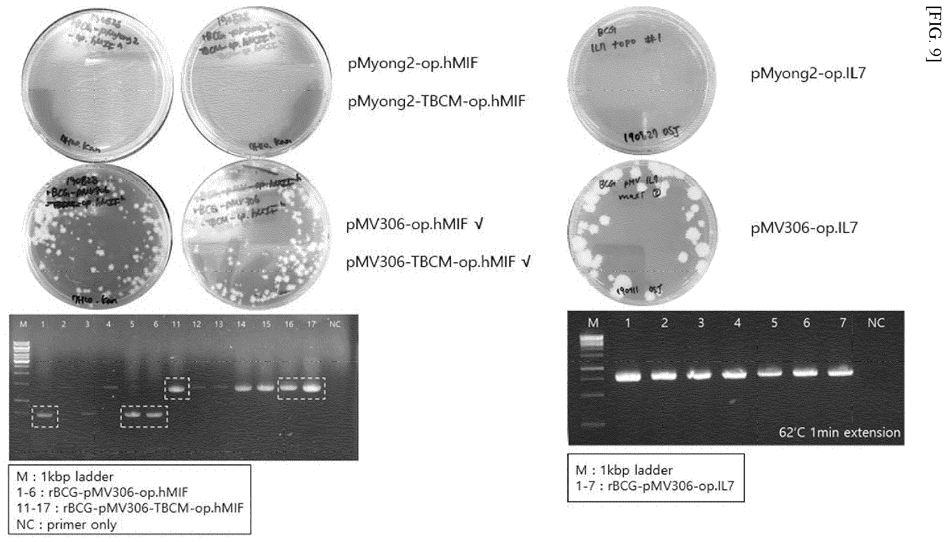

At least one strain of each recombinant *M. smegmatis* was secured and lysed by physical (ultrasonic treatment) and chemical (B-per solution containing lysosome and DNase) methods, and the proteins expressed therein were measured. As a result, it could be seen that all proteins except for hIL7 were expressed at significantly higher levels when the pMyong2 vector was used than when the pMV306 vector was used (FIG. 8). In addition, as positive controls, recombinant strains expressing various proteins in pMV306 and pMyong2 vectors were also constructed in *M. bovis* BCG (FIG. 9).

### Evaluation of stability of M smegmatis pMyong2-hMIF::IL7

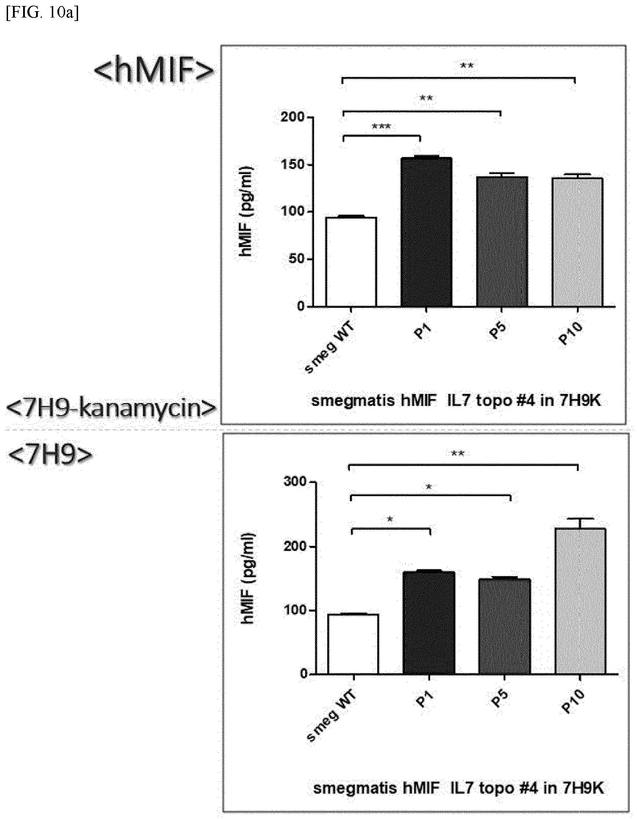

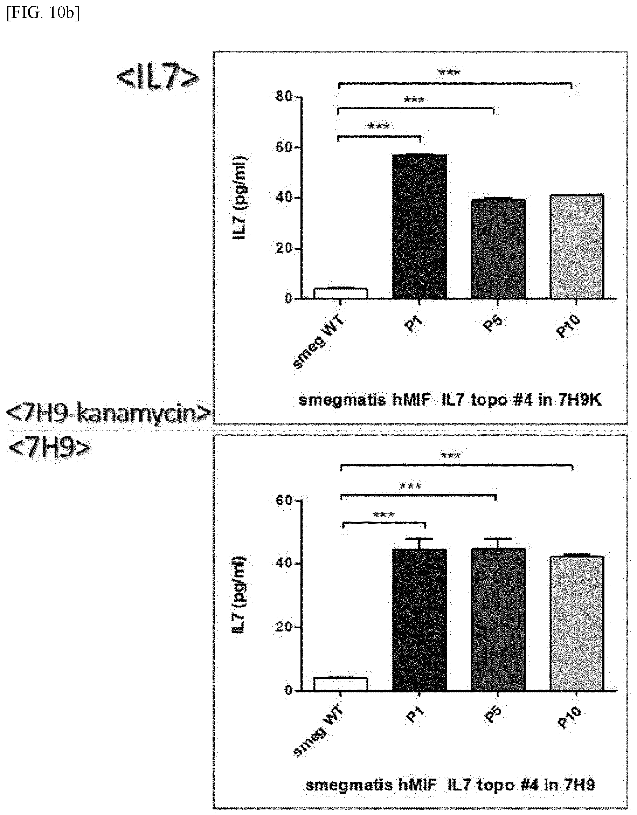

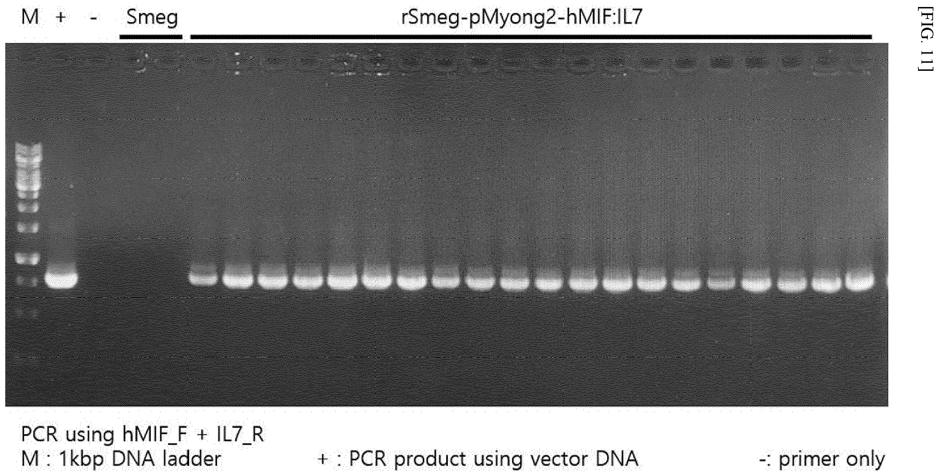

In order to evaluate the stability of the proteins expressed by *M. smegmatis* pMyong2-hMIF::IL7, the strain was cultured up to 10 passages in a medium with or without antibiotics. As a result, it was confirmed that the expression of all proteins expressed in the pMyong2 vector was maintained up to 10 passages (FIG. 10), suggesting that the recombinant strain containing the pMyong2 vector stably expressed the proteins. In addition, in order to examine whether *M. smegmatis* pMyong2-hMIF::IL7 stably expresses the proteins even in an *in vivo* environment where antibiotic selection is impossible, the strain was injected intravenously into mice, and after one week, single bacteria in the spleen were examined by PCR method. As a result, it was confirmed that *M. smegmatis* pMyong2-hMIF::IL7 stably retained the plasmid vector even in the *in vivo* environment (FIG. 11).

### Evaluation of safety of recombinant strains

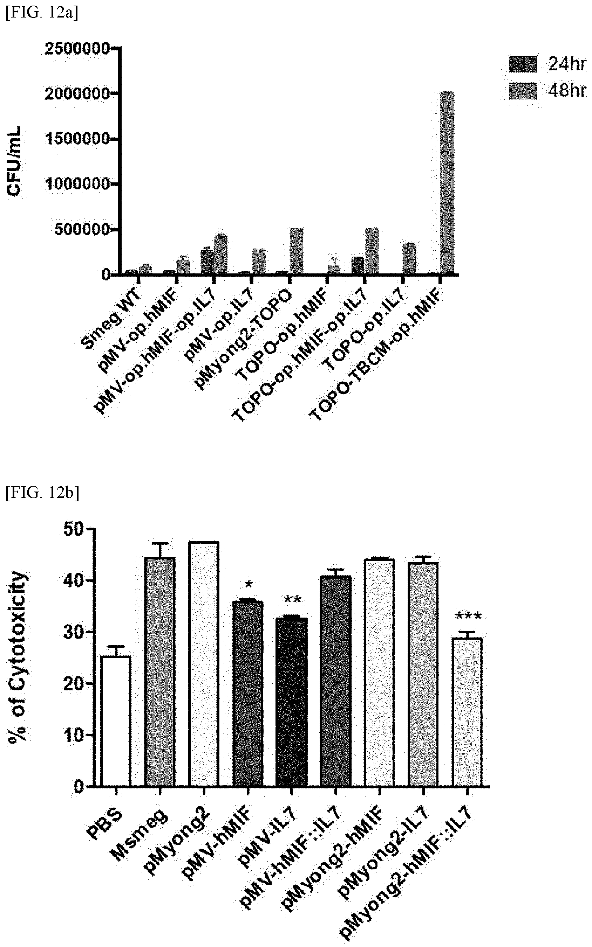

In order to examine whether the recombinant strain of the present invention is safe as a therapeutic vaccine, macrophages were infected with each recombinant strain, and at 24 and 48 hours after infection, the CFU of the recombinant strain was measured. As a result, it was confirmed that, at 24 hours after infection, the pMV306-hMIF::hIL7 and pMyong2-hMIF::hIL7 strains had higher infectivity than wild-type *M. smegmatis,* and at 48 hours after infection, all of the recombinant strains had higher infectivity than wild-type *M. smegmatis* (FIG. 12a). In addition, as a result of measuring lactate dehydrogenase (LDH), which is an enzyme present in most cells and is secreted when cells are damaged or destroyed, in the culture of the infected macrophages, it was confirmed that the cytotoxicity of the recombinant strains expressing pMV306-hMIF, -hIL7 and pMyong2-hMIF::IL7 was significantly lower than that of wild-type *M. smegmatis* with low pathogenicity, and when the macrophages were infected with *M. smegmatis* expressing pMyong2-hMIF::IL7, cytotoxicity similar to that of the PBS group not infected with any bacteria was observed (FIG. 12b). This suggests that the recombinant strains do not have high cytotoxicity despite their high infectivity for macrophages compared to the wild-type *M. smegmatis.* In particular, since the rSmeg-pMyong2-hMIF::hIL7 strain showed high cell infectivity, it was expected that the activation of immune cells would be induced by the proteins expressed by the recombinant strain, but infection with rSmeg-pMyong2-hMIF::hIL 7 did not induce the death of immune cells. Thus, it was judged that rSmeg-pMyong2-hMIF::hIL7 would show advantages optimized for use as a therapeutic vaccine.

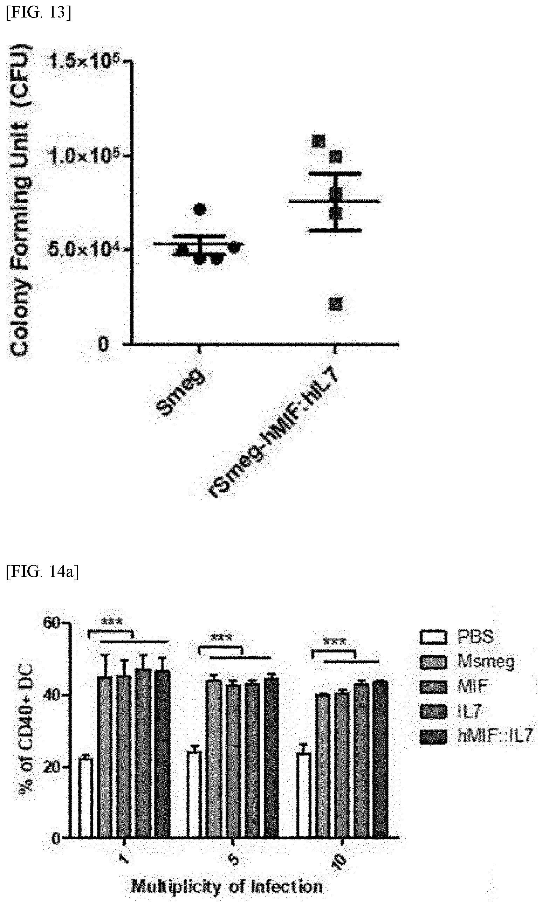

In order to examine organ infectivity of *M. smegmatis* pMyong2-hMIF::IL7 compared to the wild-type an *in vivo* environment, the strain was intravenously injected into mice (5 x 10⁶ in 100 µl PBS), and one week after injection, the spleens were homogenized and CFU was compared. As a result, it was confirmed that the recombinant strain had no statistical significance compared to the wild-type (p = 0.1887) (FIG. 13).

### Evaluation of immune enhancing ability of recombinant M. smegmatis

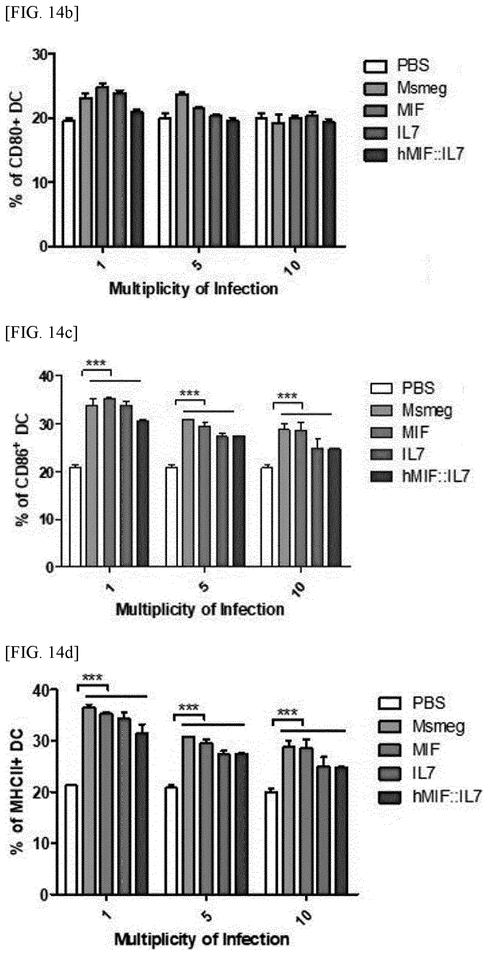

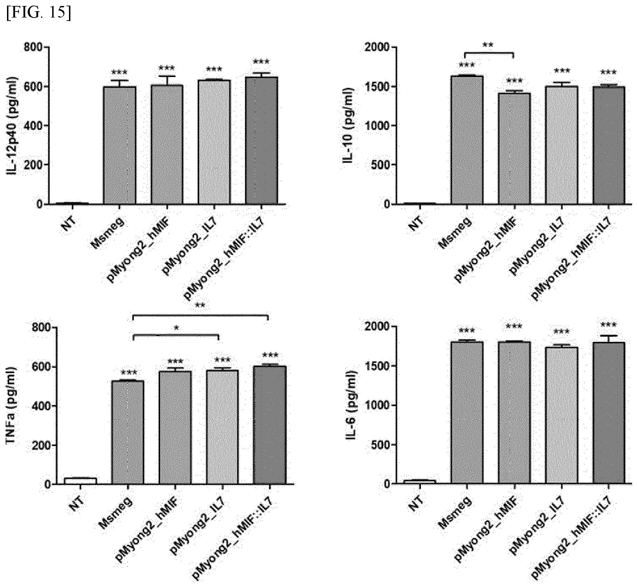

Since dendritic cells that activate acquired immunity act as important cells to induce an immune response to enhance anticancer effects, the present inventors examined whether pMyong2 recombinant *M. smegmatis* would induce the maturation of dendritic cells, which are antigen-presenting cells. Dendritic cells differentiated from mouse bone marrow cells using GM-CSF (granulocyte macrophage colony stimulating factor) were infected with the recombinant strain, and then the expression of MHCII, CD40, CD80, and CD86, which are representative maturation markers of dendritic cells, was analyzed by FACS. As a result, it was confirmed that, when dendritic cells were infected with each of recombinant *M. smegmatis* strains and wild-type *M. smegmatis* at any M.O.I., dendritic cells expressing CD40, CD86, and MHCII significantly increased compared to untreated dendritic cells. This suggests that all of the recombinant *M. smegmatis* strains stimulated and matured dendritic cells to the level shown by wild-type *M. smegmatis* (FIG. 14). In addition, as a result of measuring the inflammatory cytokines IL-6, IL-10, IL-12, and TNF-α secreted from activated dendritic cells by ELISA, it was observed that dendritic cells infected with each of the recombinant *M. smegmatis* strains and wild-type *M. smegmatis* significantly increased compared to untreated dendritic cells (FIG. 15). This suggests that all of the recombinant *M. smegmatis* stimulate dendritic cells to induce secretion of inflammatory cytokines while maintaining the characteristics of wild-type *M. smegmatis.* In conclusion, pMyong2 recombinant *M. smegmatis* of the present invention effectively induced an immune response.

### Observation of anticancer effect against various cancer cell lines

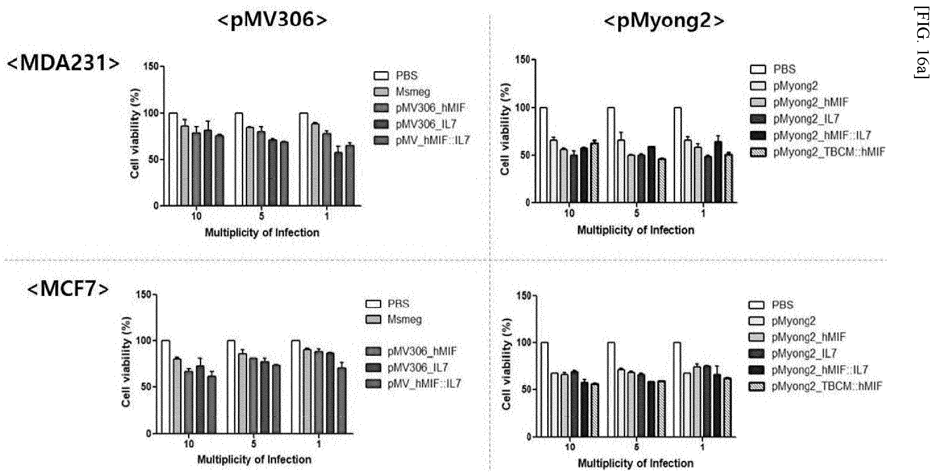

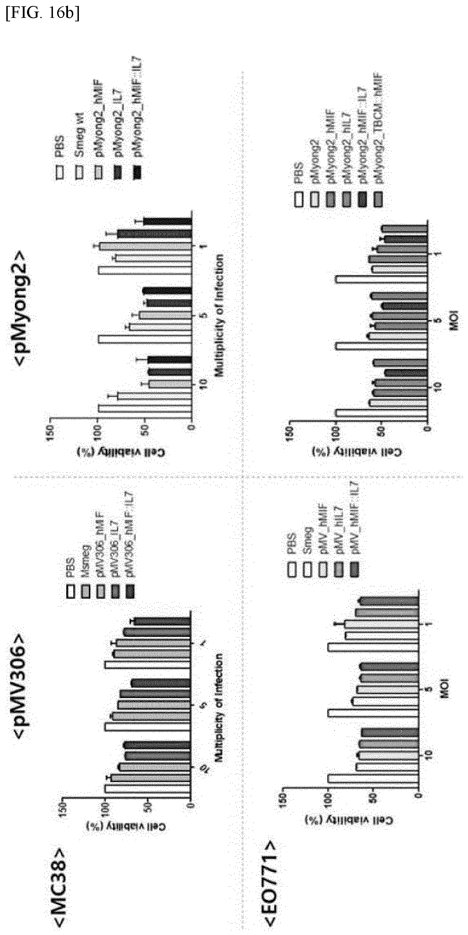

In order to evaluate the *in vitro* anticancer effect of the recombinant strains of the present invention identified above, human breast cancer cells MDA231 and MCF7, mouse colon cancer cells MC38, and mouse breast cancer cells EO771 were infected with each of the recombinant strains, and then the cell killing ability of each recombinant strain was evaluated by measuring the metabolic activity of the cancer cells. The metabolic activity of the cells was measured using the MTS cell proliferation assay kit (Promega, USA). It was confirmed that, in both human-derived cancer cells (FIG. 16a) and mouse-derived cancer cells (FIG. 16b), the recombinant strain transformed with the pMyong2 vector showed higher cytotoxicity against the cancer cells than the recombinant strain transformed with the pMV306 vector. This is presumed to be because the pMyong2-TOPO vector has significantly higher expression levels of human MIF and human IL-7 than the pMV306 vector, as confirmed in the performance of the recombinant strain observed above. In addition, it was confirmed that the strains expressing human MIF and human IL-7, respectively, tended to have higher cytotoxicity than the strain containing the empty vector, and that the case in which human MIF and human IL-7 proteins were expressed as a fusion protein showed higher cytotoxicity against cancer cells than the case in which human MIF and human IL-7 were expressed separately.

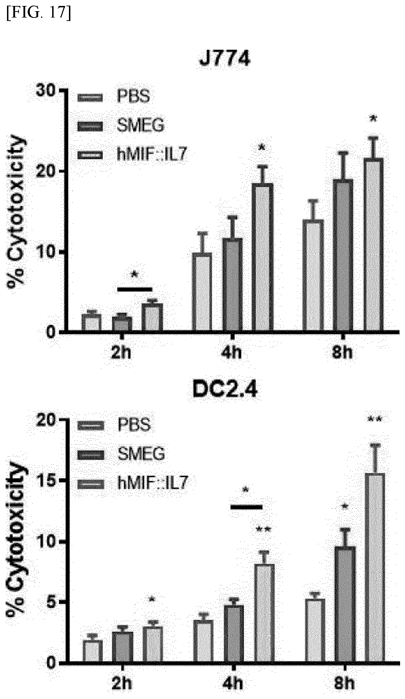

In addition, in order to examine whether macrophages and dendritic cells activated by the recombinant strain have cytotoxicity against cancer cells, macrophages and dendritic cells stimulated with the recombinant strain were co-cultured with cancer cells, and cytotoxicity against the cancer cells was evaluated. As a result, it was confirmed that the macrophages and dendritic cells stimulated by each of the recombinant strains had higher cancer cell killing effects than those stimulated with wild-type *M. smegmatis,* and in particular, pMyong2-hMIF::IL7 maximized the anticancer effect of these immune cells compared to the other recombinant *M. smegmatis* strains (FIG. 17). Thereby, it was found that the recombinant *M. smegmatis* strains were capable of inducing cancer cell death not only by directly inhibiting cancer cell metabolism, but also by stimulating macrophages and dendritic cells, and among the recombinant strains, the *M. smegmatis* strain obtained by transforming the fusion protein hMIF::IL7 into the pMyong2 vector exhibited the best anticancer effect.

### Inhibition of cancer cell migration and invasion by M. smegmatis-pMyong2 hMIF::IL7

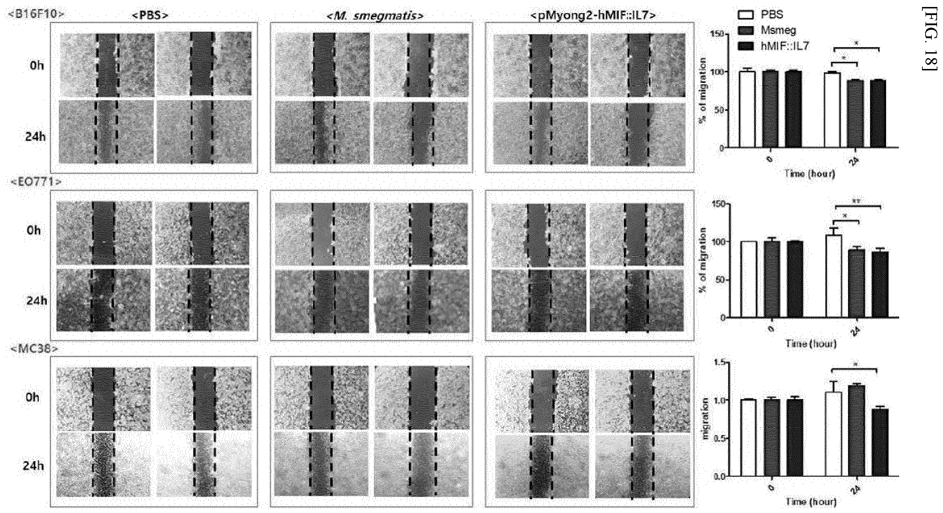

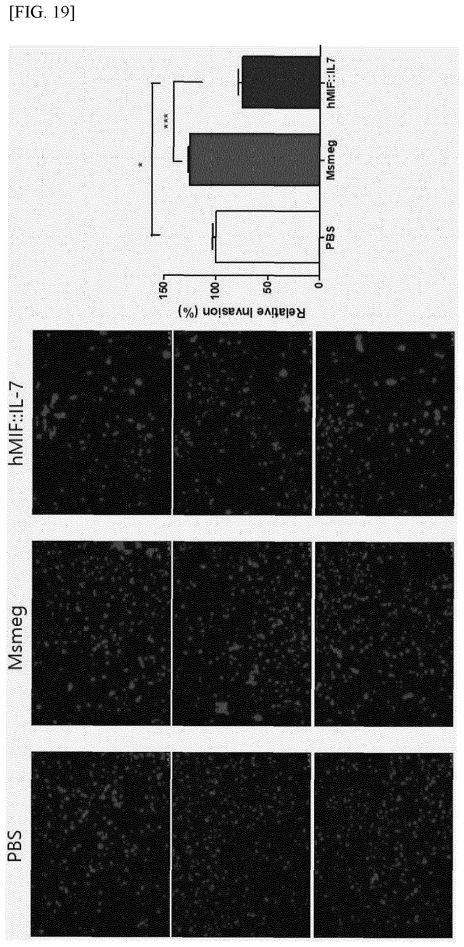

As cancer progresses, cancer cells acquire the capability to migrate and invade other tissues in order to metastasize to other organs, which causes limitations in cancer treatment. In order to examine whether *M. smegmatis*-pMyong2_hMIF::IL7 also affects migration and invasion related to cancer cell metastasis, various mouse-derived cancer cells (melanoma cells B16F10, breast cancer cells EO771, and colorectal cancer cells MC38) were infected with PBS, *M. smegmatis,* or *M. smegmatis*-pMyong2_hMIF::IL7, and then scraped with a 200 µl pipette tip, and then the degree of migration with time was checked. As a result, it could be seen that migration was inhibited in the B16F10 and EO771 cells infected with each of *M. smegmatis* and *M. smegmatis-*pMyong2_HMIF::IL7 compared to uninfected cells (FIG. 18). In particular, it was confirmed that only the *M. smegmatis*-pMyong2_hMIF::IL7 strain inhibited migration of the MC38 colorectal cancer cell line. In addition, cancer cell metastasis occurs through an invasion process in which they penetrate the blood vessel wall by damaging the extracellular matrix (ECM) using enzymes. For this reason, to investigate the inhibition of invasion by *M. smegmatis-*pMyong2_hMIF::IL7, the MC38 colorectal cancer cell line on a Matrigel-coated transwell was infected with each of PBS, *M. smegmatis,* and *M. smegmatis-*pMyong2_hMIF::IL7, and 48 hours, the cells were stained with Hoechst, and cells other than the cells that passed through the Matrigel were removed, followed by observation under a fluorescence microscope. Fluorescence microscope observation indicated that the invasion of the MC38 cells infected with *M. smegmatis-*pMyong2_hMIF::IL7 was reduced, which was statistically significant compared to when PBS orM. *smegmatis* was used (FIG. 19). This suggests that the *M. smegmatis*-pMyong2_hMIF::IL7 strain inhibits the migration and invasion of the mouse-derived colorectal cancer cell line MC38.

### Observation of in vivo anticancer effect using MC38 mouse colorectal cancer cell line

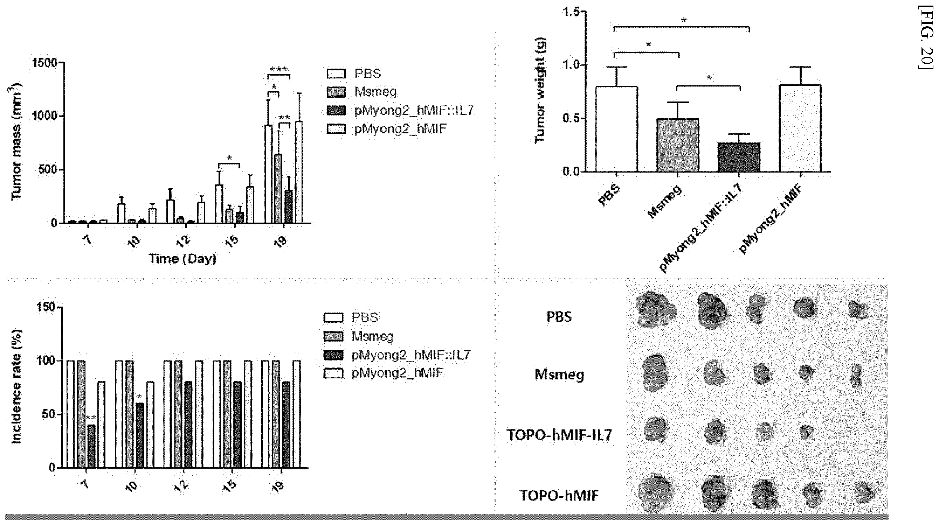

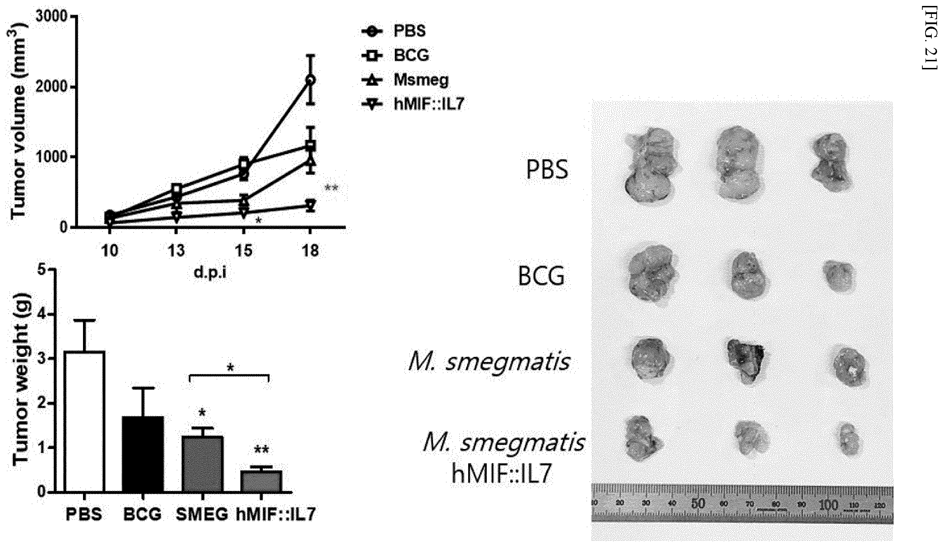

After mouse-derived colorectal cancer cells MC38 were injected into C57BL/6 mice, the recombinant strain was injected near the lymph nodes and the change in the tumor size was observed. As a result, it could be observed from 15 days after injection that the tumor size in the mice injected with *M. smegmatis* transformed with hMIF::IL7 was significantly smaller than that in the mice treated with wild-type *M. smegmatis.* It could be confirmed that, when the tumors were isolated from the mice, there was a visually distinct difference in the tumor size between the mouse groups. In addition, the weight of tumors isolated from the mice was the lowest in the case in which hMIF::IL7-transformed *M. smegmatis* was injected, and tumors were formed in the treated mice corresponding to only 80% of the untreated mice (FIG. 20). In addition, as a result of comparing the anticancer effect of *M. smegmatis*-pMyong2_hMIF::IL7 with that of BCG, which is used as a therapeutic agent for bladder cancer, the mice treated with *M. smegmatis-*pMyong2_hMIF::IL7 showed a significantly reduced tumor size compared to the BCG-treated mice from day 15 after cancer cell injection (FIG. 21). This suggests that the anticancer effect of *M. smegmatis*_pMyong2-hMIF::IL7 was not only validated *in vivo,* but also better than that of BCG, a positive control group.

### Observation of response in serum of mice with induced cancer

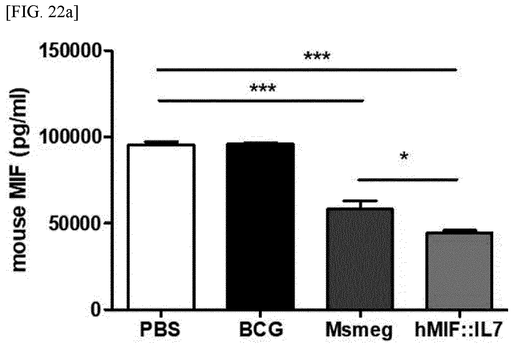

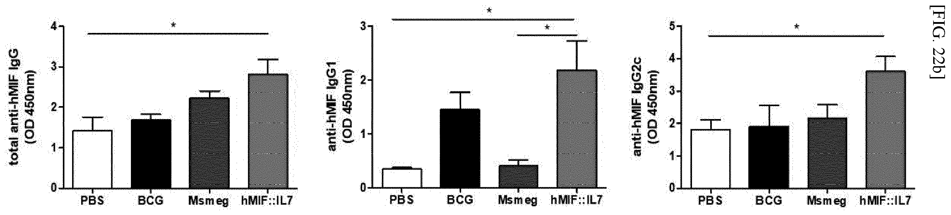

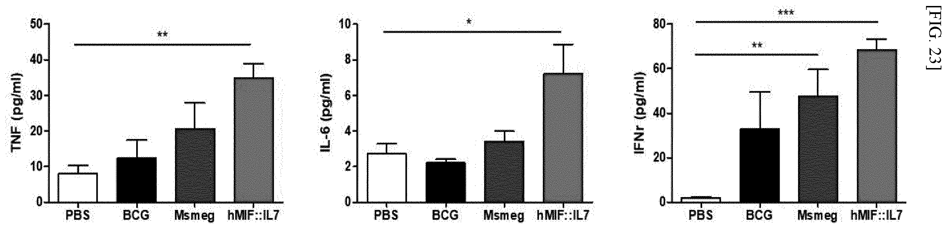

Changes in inflammatory cytokines in serum by *M. smegmatis-*pMyong2_hMIF::IL7 *in vivo* were examined by ELISA, and as a result, it could be seen that the secretion of mouse MIF in the sera isolated from the mice treated with *M. smegmatis-*pMyong2_hMIF::IL7 was significantly lower than in the sera isolated from the untreated mice and the mice treated with wild-type *M. smegmatis* (FIG. 22a). Thereby, it was determined that treatment with *M. smeginatis-*pMyong2_hMIF::IL7 would increase the concentration of IgG against MIF in the serum. In addition, it was confirmed that the concentration of anti-human MIF IgG in the sera isolated from the mice treated with *M. smegmatis*-pMyong2_hMIF::IL7 was lower than that in the sera isolated from the untreated mice and from the mice treated with wild-type *M. smegmatis* (FIG. 22b). In addition, it was confirmed that the secretion of TNF-α, IFN-γ and IL-6 in the sera isolated from the mice treated with *M. smegmatis*-pMyong2_hMIF::IL7 was significantly higher than that in the sera isolated from the untreated mice and from the mice treated with wild-type *M. smegmatis* (FIG. 23). This suggests that the overall immune activity in mice was increased by treatment with *M. smegmatis*-pMyong2_hMIF::IL7. Taken together, it was confirmed that, as the concentration of IgG against human MIF in the serum isolated from the mice treated with *M. smegmatis*-pMyong2_hMIF::IL7 was significantly higher than that in the control groups, the concentration of mouse MIF in the serum was lowered, suggesting that *M. smegmatis*-pMyong2_hMIF::IL7 efficiently induced a humoral immune response against hMIF *in vivo.*

### Observation of gene and protein expression in cancer tissues of mice with induced cancer

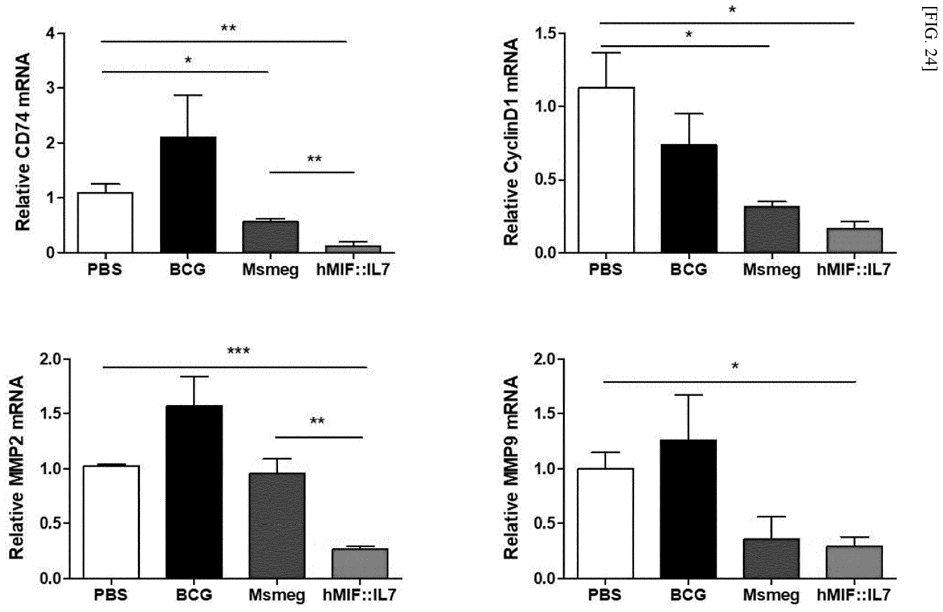

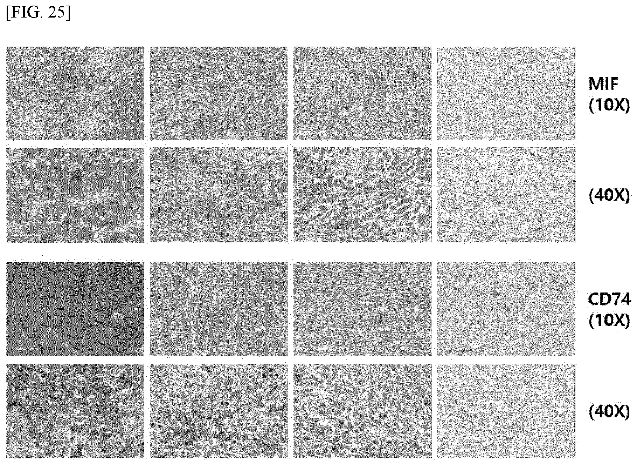

As it was confirmed that the level of anti-human MIF IgG in serum increased and the level of mouse MIF decreased in the mice injected with *M. smegmatis-*pMyong2_hMIF::IL7, whether the expression of MIF-related genes and proteins in mouse cancer tissue also decreased was examined by RT-PCR and IHC staining. As a result, it was confirmed that the mRNA expression levels of cell cycle-related Cyclin D1, CD74 (receptor for MIF), and MMP-2 and MMP-9 related to cancer growth were significantly lower in the cancer tissue of the *M. smegmatis*-pMyong2_hMIF::IL7-treated group than in the untreated mice and the wild-type *M. smegmatis*-treated mice (FIG. 24). In addition, as a result of examining protein expression by IHC staining of the cancer tissue extracted from each mouse, it was confirmed that the expression levels of CD74 (receptor for MIF) in cancer tissue and MIF protein in cytoplasm were significantly lower in the cancer tissues isolated from the *M. smegmatis*-pMyong2_hMIF::IL7-treated mice than in the untreated mice and the wild-type *M. smegmatis*-treated mice (FIG. 25). This suggests that *M. smegmatis-*pMyong2_hMIF::IL7 efficiently induces an immune response against MIF compared to BCG and wild-type *M. smegmatis*, thereby reducing the expression of genes and proteins related to cancer growth, thus inhibiting cancer growth and proliferation in the most effective manner.

### Observation of immune response in mice with induced cancer

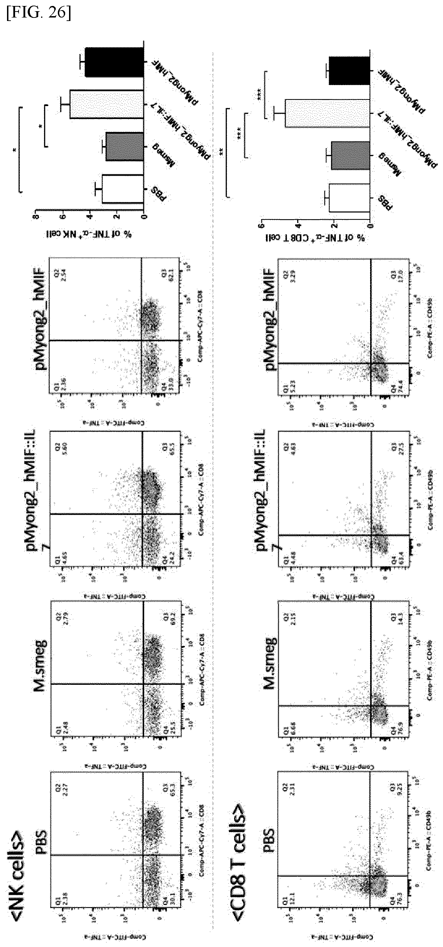

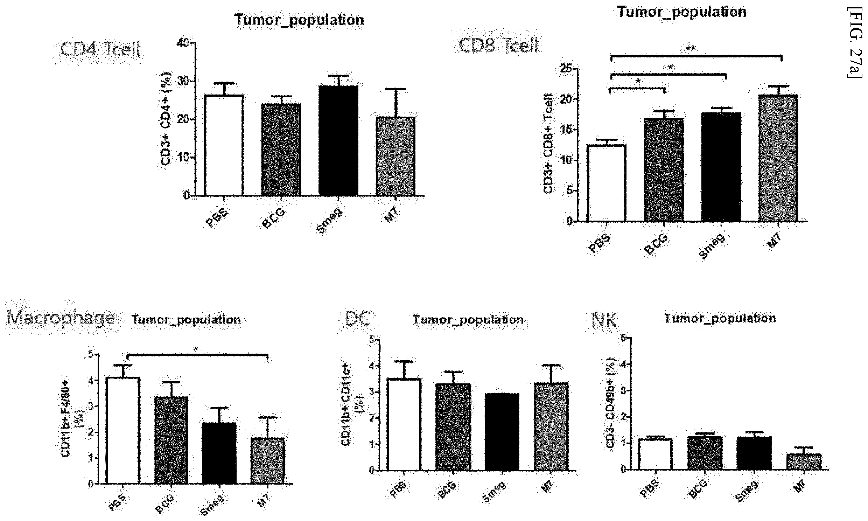

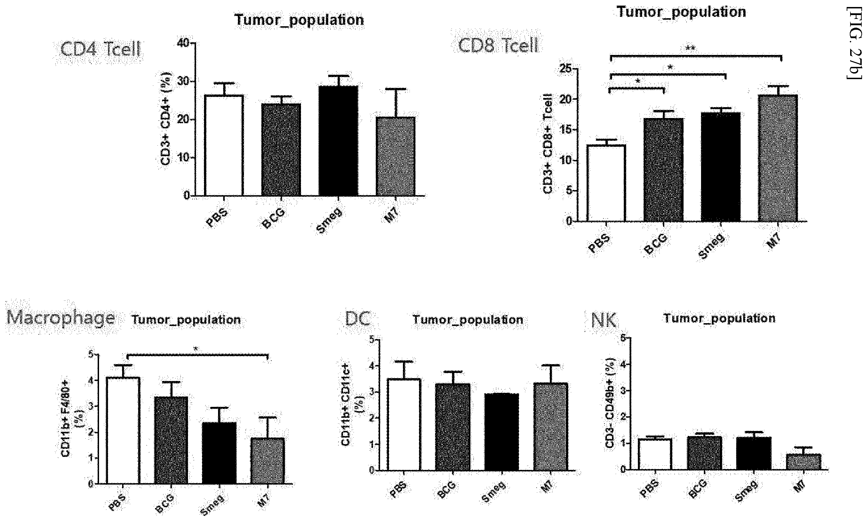

The anticancer effect observed in the first *in vivo* experiment was validated by flow cytometry, because it was determined that there would be a difference in the proportion of immune cells in the immune organ spleen as the recombinant strain was injected near the lymph node. As a result, it was confirmed that the proportions of NK and CD8 T cells secreting TNF-α in the splenocytes extracted from the *M. smegmatis*_pMyong2-hMIF::IL7-treated mice were significantly higher than those in the untreated mice or the wild-type *M. smegmatis*-treated mice (FIG. 26). In addition, in the second *in vivo* experiment in which the strain was directly injected into the environment surrounding the tumor, the proportions of immune cells in the spleen and the tumor were observed through flow cytometry. As a result, the proportion of macrophages in the spleen was significantly low in the *M. smegmatis*_pMyong2-hMIF::IL7-injected mice (FIG. 27a). In addition, cancer tissue was separated into single cells by treatment with collagenase IV and DNase I, and the proportion of immune cells was compared in the same manner. As a result, it was confirmed that, as in splenocytes, the proportion of macrophages in the M. *smegmatis*_pMyong2-hMIF::IL7-injected mice decreased. Interestingly, CD8 cytotoxic T cells having the ability to eliminate cancer cells were increased by *M. smegmatis*_pMyong2-hMIF::IL7 (FIG. 27b).

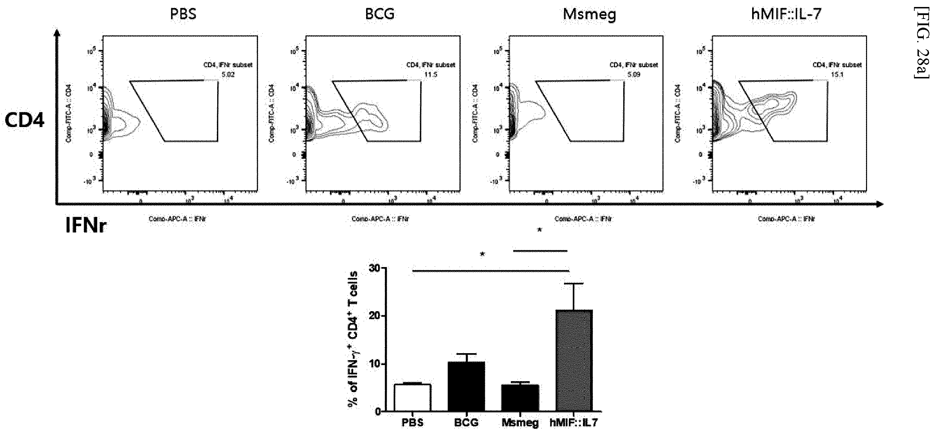

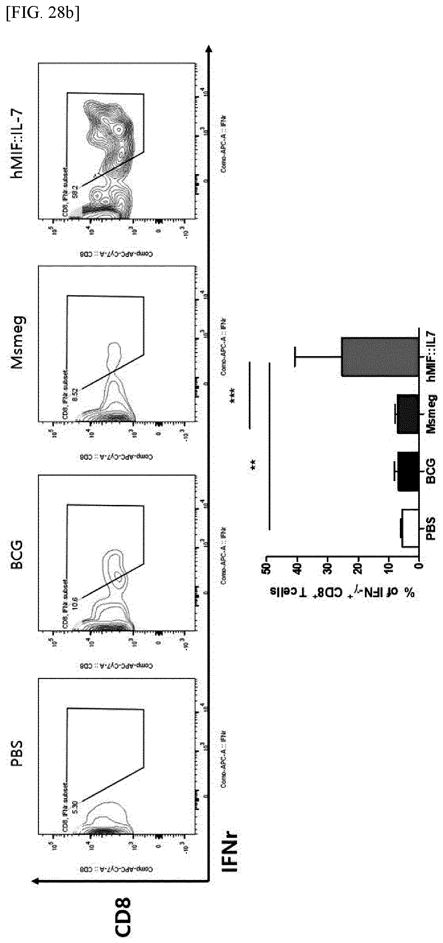

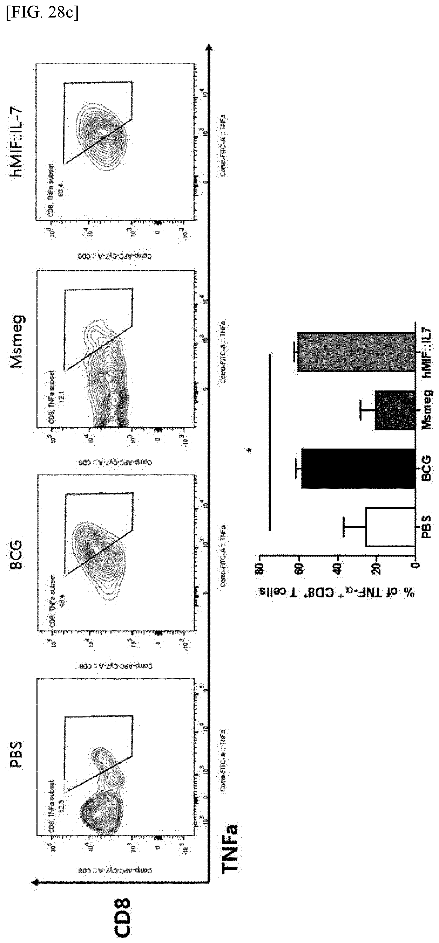

CD8 T cells that increased in tumor tissue must secrete cytokines such as TNF-α and IFN-γ in order to actually kill cancer cells or further activate their surrounding immune cells. Accordingly, flow cytometry was performed to observe whether CD8 T cells that were increased in tumor tissue by *M. smegmatis*_pMyong2-hMIF::IL7 would exert substantial anticancer function by secreting TNF-α and IFN-y, and whether CD4 T cells that activate CD8 T cells by secreting IFN-γ would be increased by *M. smegmatis*_pMyong2-hMIF::IL7. As a result, it was confirmed that the proportions of CD4 T cells and CD8 T cells secreting IFN-γ were significantly increased by *M. smegmatis*_pMyong2-hMIF::IL7, and this increase was significant compared to those in the BCG and *M. smegmatis* groups. In addition, it was confirmed that CD8 T cells secreting TNF-α that actually kills cancer cells significantly increased compared to those in the PBS and *M. smegmatis* groups (FIG. 28).

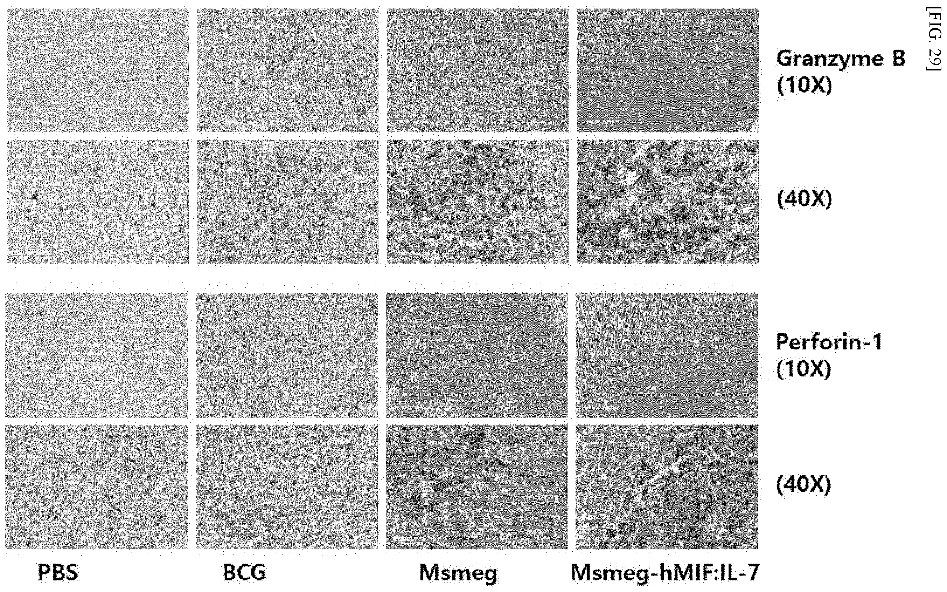

Since it was observed that splenocytes, cytotoxic T cells and Th1 cells increased, the expression of the cytolytic proteins granzyme B and perforin-1 in cancer tissue was analyzed by IHC staining. As a result, it was confirmed that the expression of granzyme B and perforin-1 proteins in the cancer tissue isolated from the mice treated with *M. smegmatis-*pMyong2_hMIF::IL7 was significantly higher than those in the untreated mice and the mice treated with wild-type *M. smegmatis* (FIG. 29). Taken together, it could be confirmed that *M. smegmatis-*pMyong2_hMIF::IL7 exhibited better anticancer effects than BCG and wild-type *M. smegmatis* by activating immune cells that induce the secretion of inflammatory cytokines in cancer tissue and spleen cells. This can also be confirmed through the expression of cytolytic proteins in cancer tissue.

### Evaluation of effect of co-administration with cisplatin

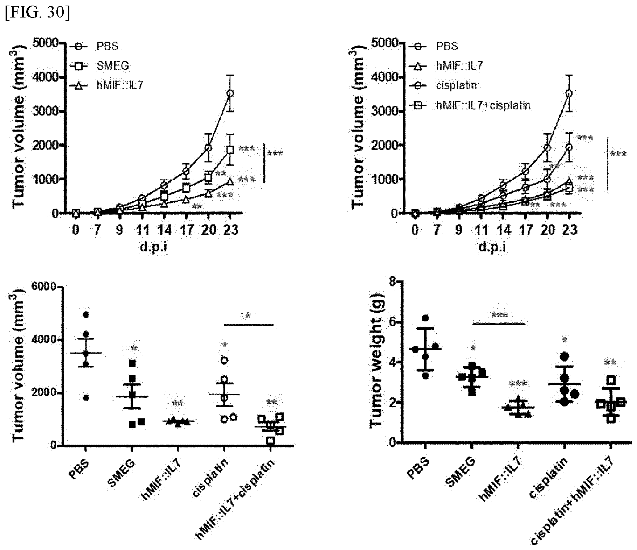

To observe the effect of co-administration of *M. smegmatis-*pMyong2_hMIF::IL7 and cisplatin, a commercially available anticancer drug, in a mouse model transplanted with the MC38 cancer cell line, each of *M. smegmatis* and *M. smeginatis*-pMyong2_HMIF::IL7 (2 x 10⁶) was injected on days 3, 7 and 14 after injection of MC38 cancer cells, and cisplatin was also injected on days 7 and 14. As a result, it could be confirmed that the growth rate of tumor volume decreased in the mice injected with each of *M. smegmatis* and *M. smegmatis*-pMyong2_hMIF::IL7 compared to the untreated mice, and that hMIF::II,7-expressing *M. smegmatis* delayed the growth rate of cancer tissue compared to the wild type. In addition, it was confirmed that the weight of the harvested cancer tissue decreased at a statistically significant level in the group injected with each of *M. smegmatis* and *M. smeginatis*-pMyong2_hMIF::IL7 compared to the untreated group, and that *M. smegmatis* expressing the hMIF::II,7 protein reduced the weight of the tumor compared to that in the wild-type treated group (FIG. 30). Accordingly, co-administration of cisplatin and *M. smegmatis*-pMyong2_hMIF::IL7 exhibited a significantly increased inhibitory effect on cancer growth compared to the administration of the anticancer drug alone.

### Measurement of mouse body weight and spleen weight when co-administered with cisplatin

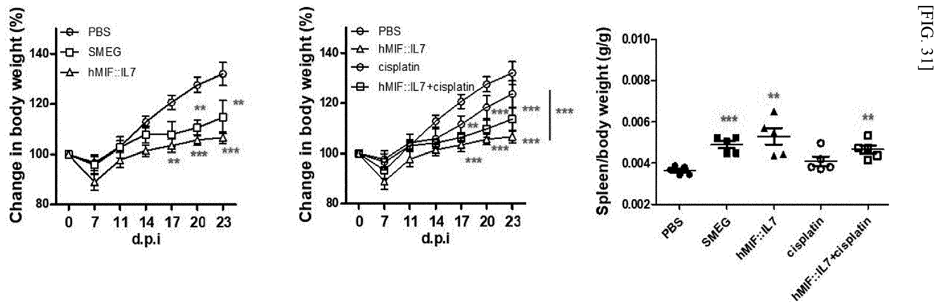

To further evaluate the effect of co-administration with cisplatin in the MC38 cancer cell line-transplanted model, the mouse weight, which is an indicator proportional to the density of cancer, and the spleen weight, which reflects the infiltration and activation of immune cells, were measured. As a result, it was confirmed that the body weight of the untreated mice steadily increased over time after cancer cell injection, whereas the body weight of the mice injected with each of *M. smegmatis* and *M. smegmatis-*pMyong2_hMIF::IL7 decreased (FIG. 31a). It was confirmed that co-administration of cisplatin and *M. smegmatis-*pMyong2_hMIF::IL7 resulted in more body weight loss compared to administration of the anticancer drug alone. It was confirmed that the spleen size increased in the mice injected with each of *M. smegmatis* and *M. smegmatis-*pMyong2_hMIF::IL7 compared to the untreated mice when viewed visually, the spleen weight versus the body weight also increased in the injected mice, and the spleen weight versus the body weight significantly decreased in the cisplatin co-administered group. Taken together, it is considered that the infiltration of immune cells into the spleen was induced by co-administration of cisplatin and *M. smegmatis-*pMyong2_hMIF::IL7.

### Changes in serum MIF concentration when co-administered with cisplatin

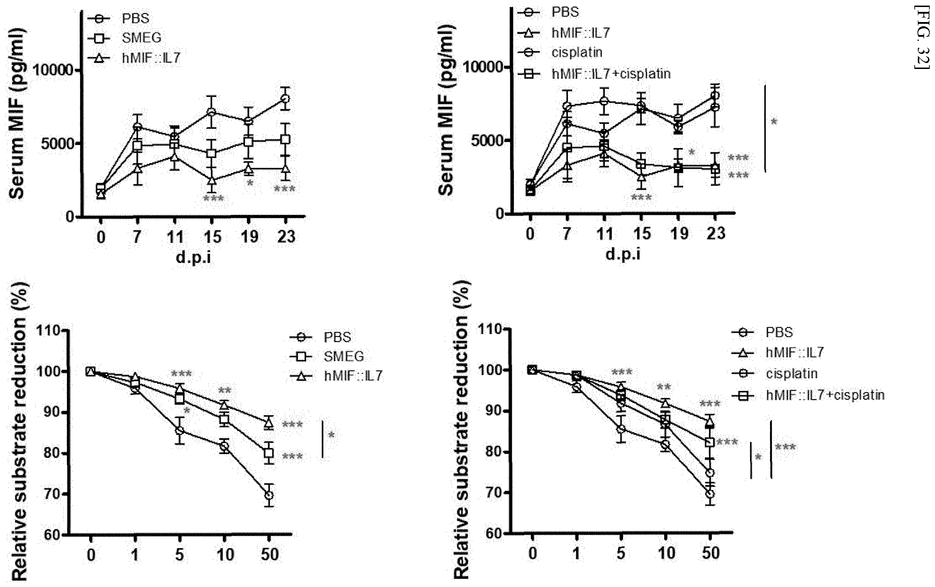

In order to further evaluate the effect of co-administration with cisplatin in the MC38 cancer cell line-transplanted model, serum was isolated through intraorbital blood collection every 4 days after cancer cell injection, and changes in MIF concentration in the serum were measured by tautomerase assay. As a result, it was confirmed that, in the case of untreated mice, the serum MIF concentration steadily increased after cancer cell injection, whereas in the case of the group treated with *M. smegmatis-*pMyong2_hMIF::IL7, the serum MIF concentration decreased at a statistically significant level (FIG. 32a).

It was confirmed that the serum MIF concentration also decreased in the group to which cisplatin, anti-PD-L1 and *M. smegmatis*-pMyong2_hMIF::IL7 were co-administered together compared to the untreated group, and that co-administration of cisplatin and *M. smegmatis*-pMyong2_hMIF::IL7 further decreased the serum MIF concentration at a statistically significant level compared to administration of cisplatin alone (FIG. 32a).

In addition, in order to evaluate the biological activity of serum MIF, the serum was diluted in a medium at concentrations of 0, 1, 5, 10, and 50%, and tautomerase assay was performed. As a result, it was confirmed that the conversion rate for substrate reduction was higher in the group treated with each of *M. smegmatis* and *M. smegmatis-*pMyong2_hMIF::IL7 than in the untreated group, and that *M. smegmatis-*pMyong2_hMIF::IL7 showed a higher degree of substrate reduction than wild-type *M. smegmatis.*

In addition, the results of tautomerase assay indicated that the conversion rate for serum substrate reduction was higher in the group to which cisplatin and *M. smegmatis*-pMyong2_hMIF::IL7 were co-administered than in the untreated group as well as the group to which cisplatin was administered alone. Thereby, it could be seen that the concentration and biological activity of serum MIF in the cancerous mice decreased when cisplatin was co-administered with *M. smegmatis*-pMyong2_hMIF::IL7 compared to when each of the components was administered alone.

### Anti-MIF antibody titer in serum of cancerous mice when co-administered with cisplatin

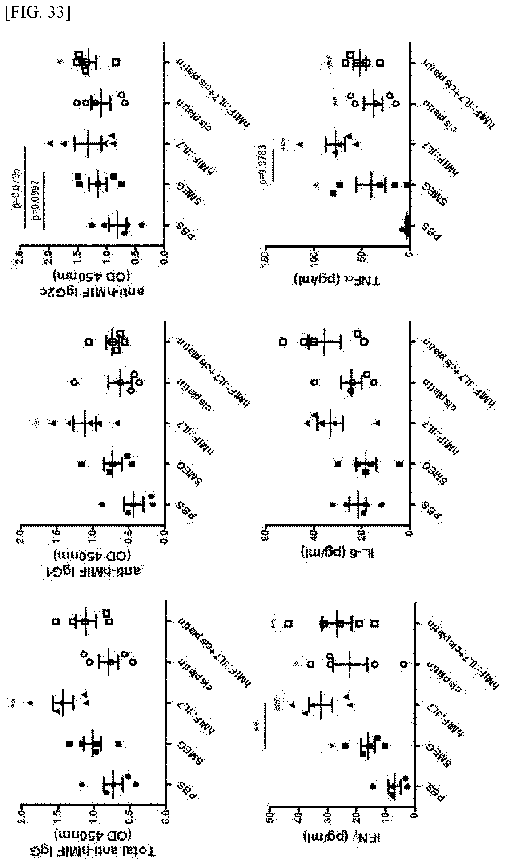

As a result of co-administration of *M. smegmatis*-pMyong2_hMIF::IL7 and cisplatin in a mouse model transplanted with the MC38 cancer cell line, the concentration and biological activity of serum MIF decreased. Thus, it was considered that the production of anti-MIF antibody increased, and it was expected that the production of serum cytokines, which is a measure of the increase in immune activity in the body, would also be increased. As a result of confirming the anti-MIF antibody titer in serum in several units, it was confirmed that, in the group treated with *M. smegmatis-*pMyong2_hMIF::IL7, the total anti-MIF IgG increased at a statistically significant level compared to that in the untreated group (FIG. 33). Serum cytokine (IFN-γ and TNF-α) levels also significantly increased in the group to which cisplatin was administered alone, the *M. smegmatis-*pMyong2_hMIF::IL7-treated group, and the group co-treated with cisplatin and *M. smegmatis*-pMyong2_hMIF::IL7, compared to the control group (FIG. 33).

### Observation of immune response in cancer tissues of cancerous mice when co-administered with cisplatin

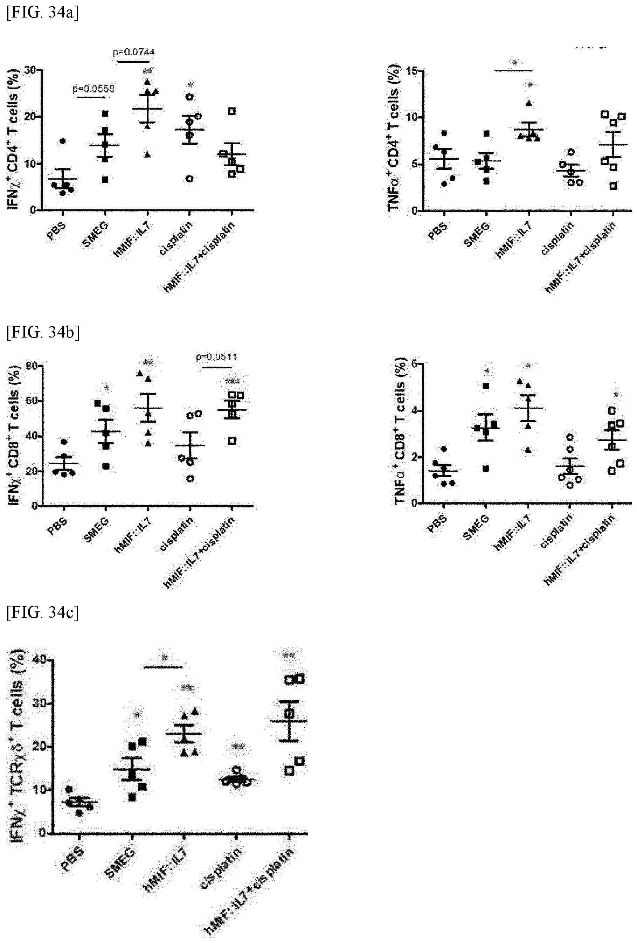

To examine whether immune cells secreting IFN-γ and TNF-α increase when a mouse model transplanted with the MC38 cancer cell line is co-treated with cisplatin and *M. smegmatis*-pMyong2_hMIF::IL7, cancer tissue was harvested on day 23 after cancer cell injection, and then separated into single cells by treatment with collagenase IV and DNase I and then passage through a strainer. Thereafter, cytokines in cancer cells were accumulated by treatment with PMA and ionomycin, followed by incubation with a fluorescently conjugated antibody, and immune cells were observed by flow cytometry. As a result, it was confirmed that the infiltration of Thi helper T cells and cytotoxic T cells that produce IFN-γ and TNF-α in cancer tissues was higher in the *M. smegmatis-*pMyong2_hMIF::IL7-treated mice, the cisplatin-treated mice, and the mice co-treated with *M. smeginatis-*pMyong2_hMIF::IL7 and cisplatin than in the untreated mice (FIGS. 34a and 34b). The proportion of TCRγδ T cells secreting IFN-γ in cancer tissue also increased at a statistically significant level in the mice treated with each of *M. smegmatis* and *M. smegmatis*-pMyong2_hMIF::IL7 compared to the control group, and the infiltration of TCRγδ T cells also increased in the cancer tissues of the cisplatin-administered mice and the mice co-treated with cisplatin and *M. smegmatis-*pMyong2_hMIF::IL7 (FIG. 34c). Thereby, it could be seen that, compared to wild-type *M. smegmatis*, *M. smegmatis*-pMyong2_hMIF::IL7 enhanced the anticancer immune response by increasing the production of TCRγδ T cells that mature Thi helper T cells and cytotoxic T cells and their infiltration into cancer tissues.

### Example 2

### Evaluation of anticancer effect against additional cancer cell lines

In order to observe the anticancer effect of *M. smegmatis-*pMyong2_hMIF::IL7 on additional various cancer cell lines other than the MC38 cancer cell line, in the same manner as the experiment using MC38, the mouse pancreatic cancer cell line PanO2 and the mouse lung cancer cell line LLC were subcutaneously injected into the upper thighs of mice, and then *M. smegmatis*-pMyong2_hMIF::IL7 was injected peritumorally a total of three times on days 3, 7 and 14, followed by observation of the tumor size.

From days 21 and 24 after injection of the PanO2 and LLC cancer cells, it could be observed that the tumor size significantly decreased in the mice injected with *M. smegmatis*-pMyong2_hMIF: :IL7 compared to the untreated mice. When the tumors were isolated from the mice, there was a visually distinct difference in the tumor size between the mouse groups.

In the same manner as the experiment using MC38, additional experiments were conducted using the sera, splenocytes, and tumors isolated from tumor-transplanted mouse models generated using PanO2 and LLC. In addition, flow cytometry was performed on activated immune cells in the tumor and spleen tissue in order to examine whether the activity of the immune cells infiltrated into the tumor increased due to the increased immune response against MIF.

Production of IgG against human MIF was analyzed using the sera isolated from the tumor-transplanted mouse models generated using PanO2 and LLC to examine the humoral immune response against MIF, and the resulting MIF concentration in the serum was analyzed. In addition, it was confirmed that, when the splenocytes were separated into single cells by removing erythrocytes therefrom and then cultured with each cancer cell lysate antigen and MIF antigen protein, inflammatory cytokines were induced.

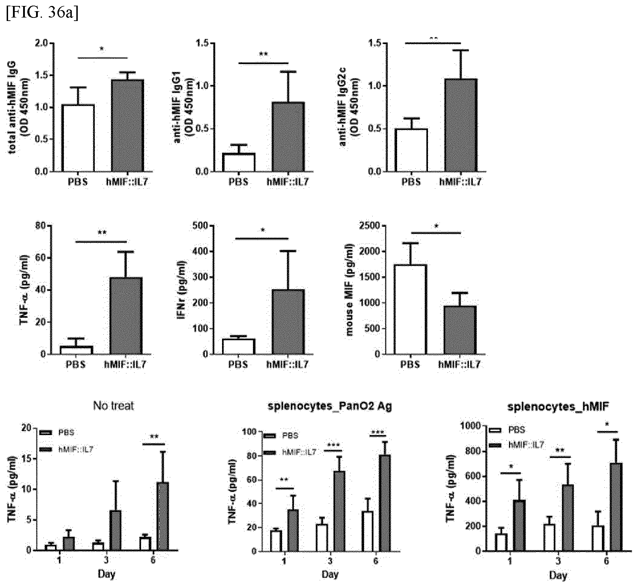

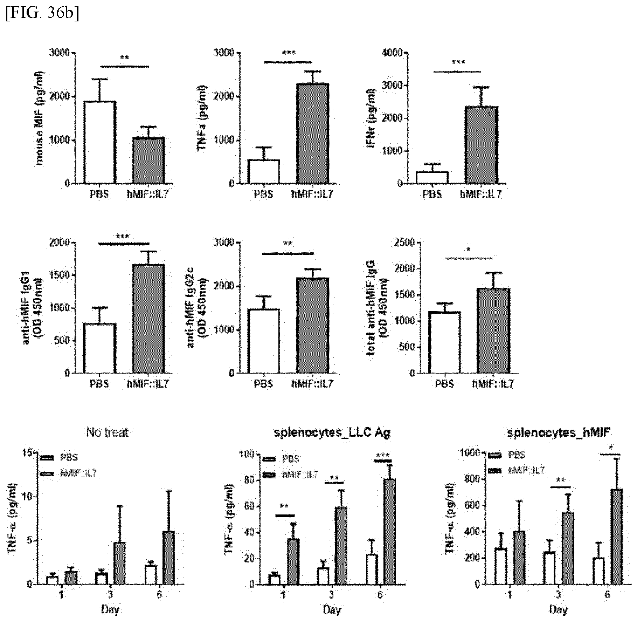

The level of IgG against human MIF in the serum isolated from the PanO2- or LLC-transplanted mouse model was higher in the *M. smegmatis*-pMyong2_hMIF::IL7-treated mice than in the untreated mice, and the level of mouse MIF in the serum was lower. Thereby, it could be seen that, when *M. smegmatis*-pMyong2_hMIF::IL7 was injected into the mouse, the humoral immune response against MIF in the mouse body increased and the serum MIF level decreased (FIG. 36).

When the splenocytes isolated from the PanO2- or LLC-transplanted mouse model were cultured with tumor cell antigen and human antigen protein, cellular immune responses against the tumor cells and MIF antigen increased in the *M. smegmatis-*pMyong2_hMIF::IL7-treated mice compared to the untreated mice (FIG. 36).

Since it was confirmed that the humoral and cellular immune responses against MIF in the PanO2-transplanted mouse model were increased by treatment with *M. smegmatis*-pMyong2_hMIF::IL7, the activated immune cells in the tumor and spleen tissues were measured by flow cytometry in order to examine whether the activity of immune cells infiltrated into the tumor increased due to the increased immune response against MIF.

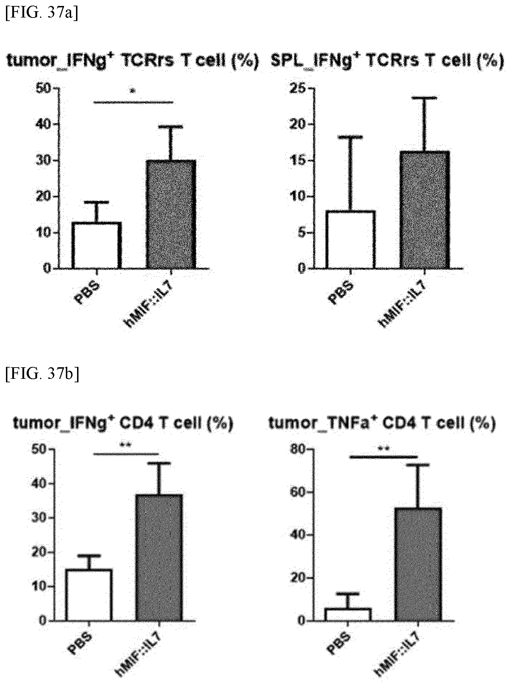

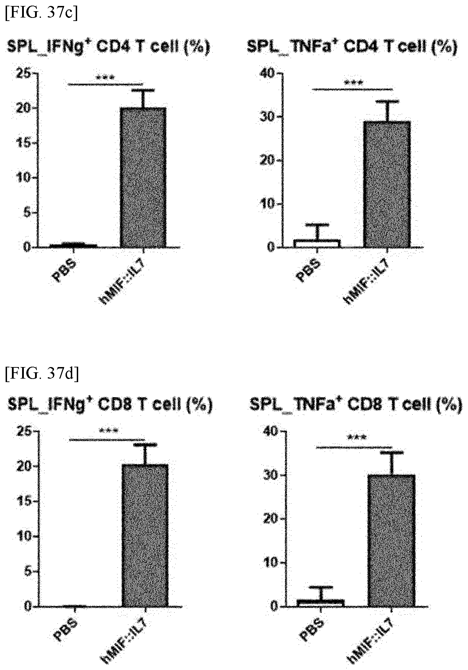

The proportion of IFNy-secreting TCRγδ T cells infiltrating the tumor tissue and spleen tissue was increased by treatment with *M. smegmatis*-pMyong2_hMIF::IL7. IFNy-secreting TCRγδ T cells are a subset that induces T cell maturation and activation, and IFNy-secreting TCRγδ T cells that increased by treatment with *M. smegmatis-*pMyong2_hMIF::IL7 were expected to induce activation of other T cells (FIG. 37a).

In the PanO2-transplanted mouse model, IFNy-secreting CD4 helper T cells and TNFα-secreting CD4 helper T cells increased in the tumor and spleen tissues of the *M. smegmatis-*pMyong2_hMIF::IL7-treated mice compared to the untreated mice (FIGS. 36b and 36c). In addition, CD4 helper T cells and cytotoxic CD8 T cells secreting IFNy and TNFα in the spleen tissue increased in the *M. smegmatis*-pMyong2_hMIF::IL7-treated mice (FIG. 36d).

These results indicate that IFNy-secreting TCRγδ T cells that induce T cell maturation and activation in the PanO2-transplanted mice were increased by injection of *M. smegmatis*-pMyong2_hMIF::IL7 injection, and for this reason, CD4 helper T cells secreting IFNy and TNFα, cytokines that can directly kill tumor cells increased in the tumor and spleen tissues.

It was confirmed that, when *M. smegmatis*-pMyong2-hMIF::IL7 was injected into the LLC-transplanted mouse model, both humoral and cellular immune responses against human MIF increased. In order to examine whether the activity of immune cells infiltrating the tumor increased due to the increased immune response against MIF, activated immune cells in the tumor and spleen tissues were measured by flow cytometry.

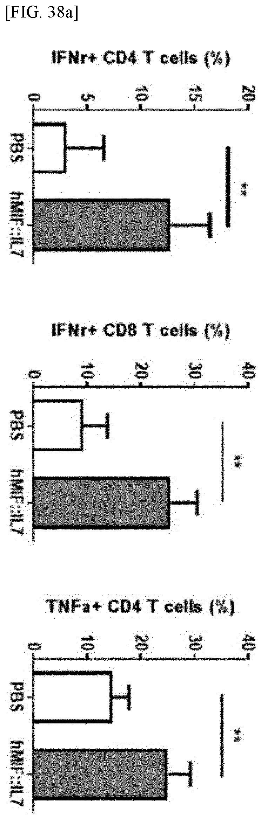

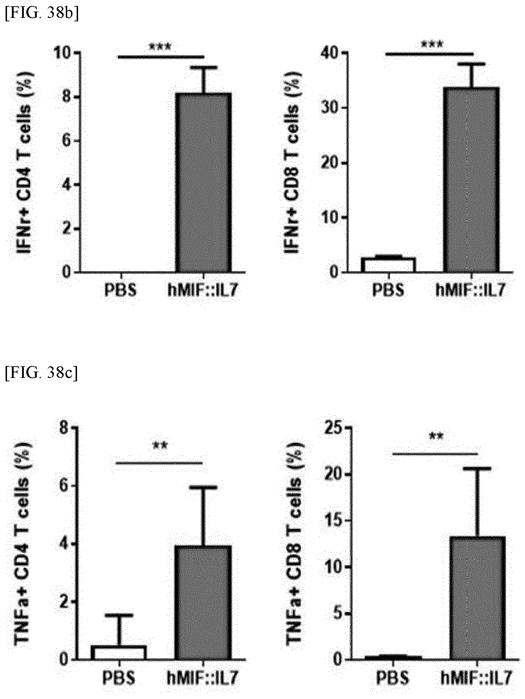

IFNy-secreting CD4 helper T cells, cytotoxic CD8 T cells, and TNFα-secreting CD4 helper T cells that infiltrated into the tumor tissue were increased by treatment with *M. smegmatis-*pMyong2_hMIF::IL7 (FIG. 38a), and also CD4 helper T cells and cytotoxic CD8 T cells secreting IFNy and TNFα in the spleen tissue also increased in the *M. smegmatis*-pMyong2_hMIF::IL7-treated mice (FIGS. 38b and 38c).

These results indicate that CD4 helper T cells and cytotoxic CD8 T cells secreting the cytokines IFNy and TNFα capable of directly killing tumor cells in the LLC-transplanted mice were increased in the tumor tissue and spleen tissue by injection of *M. smegmatis*-pMyong2_hMIF::IL7 injection. Thereby, it could be confirmed that the increase in immune response against MIF and the increase in activated immune cells in tumor and spleen tissues by injection of M. smegmatis-pMyong2_hMIF::IL7 injection, as confirmed in the MC38-transplanted mouse model, also exhibited the same anticancer effect on various cancer cell lines such as PanO2 and LLC.

### Evaluation of MIF activity inhibitory ability of serum isolated from MC38-transplanted mouse model

The MIF activity inhibitory ability of *M. smegmatis-*pMyong2_hMIF::IL7 was evaluated using serum isolated from a mouse model transplanted with MC38 cancer cell line. 48 and 72 hours after the serum isolated from the mouse was added to 50% DMEM medium for MC38 cell line culture, expression of CD74, a MIF receptor on the surface, was analyzed by flow cytometry. In addition, at 24 hours of culture in the same experiment, the expression of MIF downstream signaling protein in cells was measured by Western blot analysis, and cell growth inhibition by inhibition of MIF activity was investigated through 7AAD/Annexin V apoptosis assay.

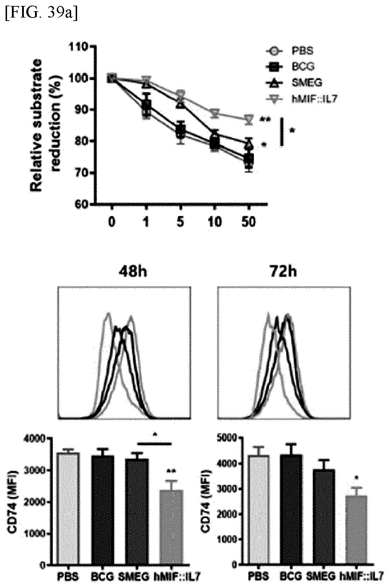

When the degree of biological activity of serum MIF was measured through tautomerase assay, it was confirmed that the activity of MIF decreased in the serum of the *M. smeginatis*-pMyong2_hMIF::IL7 group compared to wild-type *M. smegmatis* (FIG. 39a).

The expression of CD74, a MIF receptor on the cell surface, in MC38 cells cultured in a medium containing serum at 48 hours and 72 hours of culture, significantly decreased in the serum isolated from the *M. smegmatis-*pMyong2_hMIF::IL7-treated mice compared to the mice treated with each of BCG and wild-type *M. smegmatis.* This appears to be because the injection of *M. smegmatis*-pMyong2_hMIF::IL7 decreased the level of MIF in the mouse serum and increased the humoral immune response against MIF, resulting in a decrease in the amount of MIF in the culture of the MC38 cell line, resulting in inhibition of the expression of the MC38 receptor (FIG. 51a).

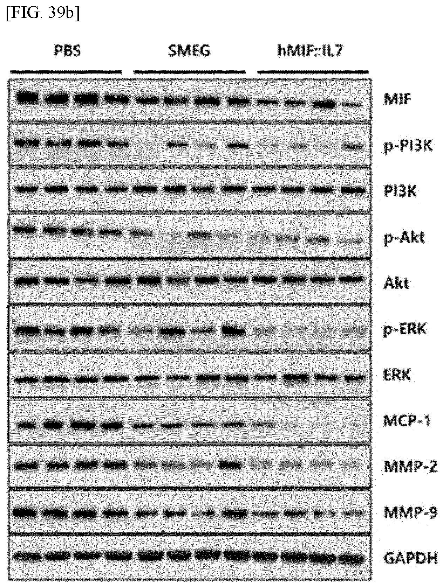

In addition, in the same experiment, treatment with the serum isolated from the *M. smegmatis*-pMyong2_hMIF::IL7 group was performed in order to examine the expression of proteins related to the proliferation and metastasis of intracellular MIF-related cancer cells. As a result, it could be observed that the expression of the proteins decreased compared to that in the wild-type *M. smegmatis*-treated group. This also suggests that the amount of MIF in the MC38 cell culture was decreased by the serum isolated from the *M. smegmatis*-pMyong2_hMIF::IL7 group, thereby inhibiting the expression of proteins related to cancer cell growth (FIG. 39b).

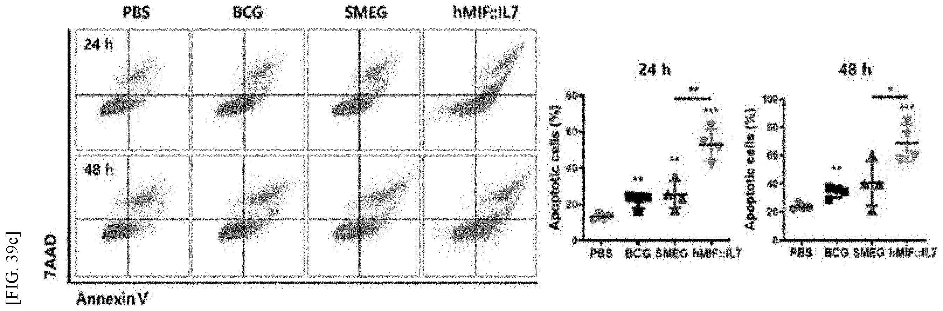

In addition, as a result of the 7AAD/Annexin V apoptosis assay, it was confirmed that the apoptotic MC38 cells increased when treated with the serum from the *M. smegmatis*-pMyong2_hMIF::IL7 group compared to when treated with each of BCG and wild-type *M. smegmatis.* Similarly, it was thought that the decrease in MIF in the cell culture due to the MIF activity inhibitory ability of the *M. smegmatis-*pMyong2_hMIF::IL7 serum would lead to increased apoptosis of MC38 cells (FIG. 39c).

These results suggest that the humoral immune response against MIF, which was increased by injection of *M. smegmatis*-pMyong2_hMIF::IL7, directly decreased the activity of MIF, thereby inhibiting the growth and survival of the cancer cells.

Since it was confirmed that injection of *M. smegmatis*-pMyong2_hMIF::IL7 increased the humoral immune response against MIF in mouse serum, the present inventors examined the ability of the MC38 cell line to migrate and invade, thereby demonstrating the ability of *M. smeginatis-*pMyong2_hMIF::IL7 to inhibit not only the growth of cancer cells but also metastasis of cancer cells. For migration assay and invasion assay, 50% and 20% sera isolated from MC38-transplanted mice were added to MC38 cell cultures, respectively, and the cells were cultured for 24 hours. As a result, it was confirmed that migration and invasion abilities of the cancer cells were significantly inhibited by the serum isolated from the *M. smegmatis*-pMyong2_hMIF::IL7-treated group compared to BCG and wild-type *M. smegmatis* (FIG. 52). This suggests that *M. smeginatis*-pMyong2_hMIF::EL7 is able to reduce the MIF activity of the MC38 cell culture, thereby inhibiting the growth and migration of the MC38 cells, thereby ultimately inhibiting cancer metastasis.

### Analysis of protein and RNA expression in tumor tissue isolated from MC38-transplanted mouse model

To measure the protein and RNA expression changes in tumor tissue by *M. smeginatis-*_hMIF::IL7 in a mouse model transplanted with MC38 cancer cell line, MC38 tumor tissue was homogenized, and then intracellular protein was extracted therefrom, and RNA was extracted from the tumor tissue using Trizol reagent. After protein quantification, the expression of proteins related to cancer cell growth and metastasis was analyzed by Western blotting, and the expression of transcription factors related to cell growth, apoptosis, angiogenesis and metastasis was analyzed by RT-qPCR after cDNA synthesis from RNA.

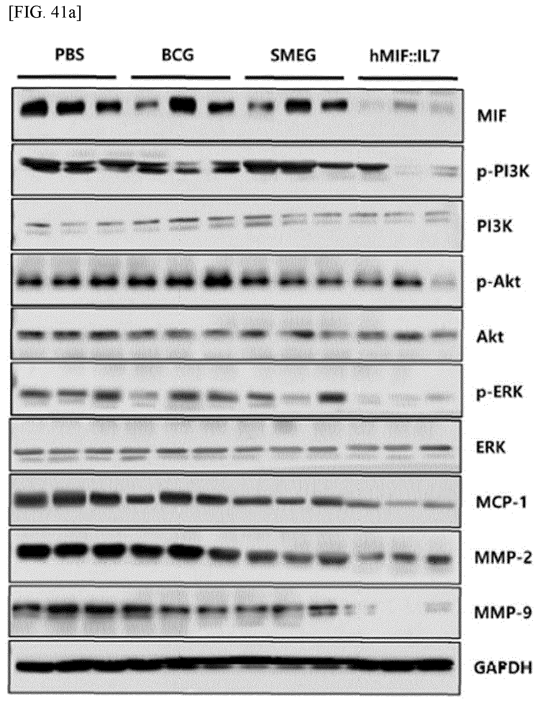

As a result of analyzing the protein expression level in tumor tissue by Western blot analysis, it was confirmed that the PI3K (phosphoinositide 3-kinase)/Akt (protein kinase B) pathway, which is directly activated by MIF, was most reduced in the *M. smegmatis*-pMyong2_hMIF::IL7-treated group, and that the expression of MMP-2 and MMP-9 proteins related to cancer cell metastasis was most reduced in the tumor tissue isolated from the *M. smegmatis*-pMyong2_hMIF::IL7-treated group (FIG. 41a).

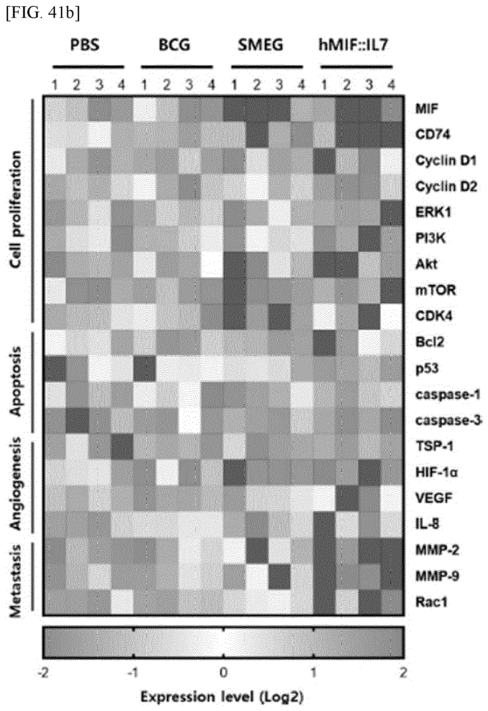

In addition, as a result of analyzing RNA expression in the tumor tissue by RT-qPCR, it was confirmed that the expression of RNA related to the PI3K/Akt pathway and cell growth and metabolism most significantly decreased in the tumor tissue isolated from the *M. smegmatis*-pMyong2_hMIF::IL7-treated group. In addition, it was confirmed that transcription factors related to apoptosis, angiogenesis and metastasis of cancer cells were most significantly reduced in the tumor tissue isolated from the *M. smegmatis-*pMyong2_hMIF::IL7-treated group (FIG. 41b).

Based on these results, it was thought that the expression of MIF-related PI3K/Akt signaling pathway protein and RNA in tumors of the MC38-transplanted mice, and the expression of RNA related to cancer cell angiogenesis were decreased by treatment with *M. smegmatis*-pMyong2_hMIF::IL7, thus inhibiting the growth of the tumor tissue.

### Anticancer effect of co-administration with anti-PD-L1

The effect of co-administration with anti-PD-L1 (anti-mouse PD-L1 B7-H1, InvivoMab, catalog# BE0101), a commercially available anticancer drug, was evaluated in a mouse model transplanted with the MC38 cancer cell line. In the same manner as described above, mycobacteria were injected on days 3, 7, and 14 after cancer cell injection, and anti-PD-L1 was intraperitoneally injected twice on days 7 and 14 after cancer cell injection. Then, the size of the tumor tissue, the weight of the extracted tumor tissue, and the change in the serum MIF level were measured.

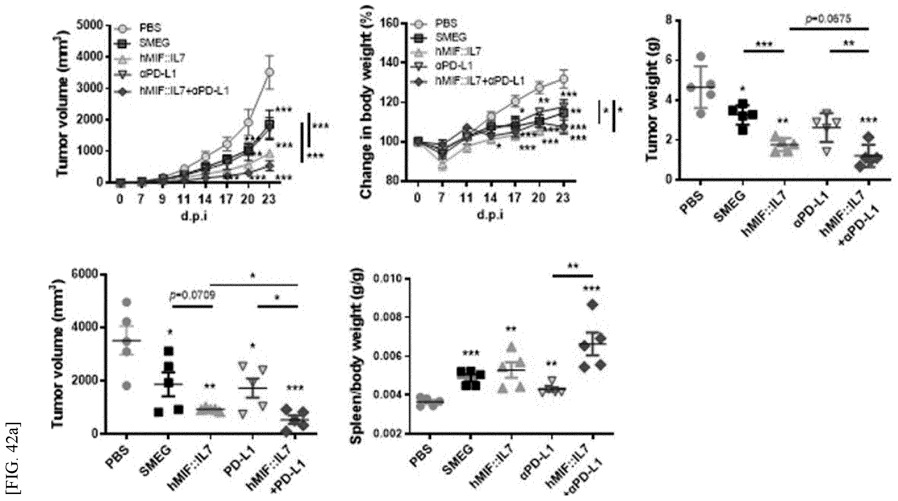

As a result, it was confirmed that cancer growth was significantly inhibited when co-administered with anti-PD-L1 compared to when M. smegmatis-pMyong2_hMIF::IL7 was administered alone, and the tumor weight at the time of cancer extraction also significantly decreased when *M. smegmatis*-pMyong2_hMIF::IL7 and anti-PD-L1 were co-administered compared to when they were administered alone (FIG. 42a).

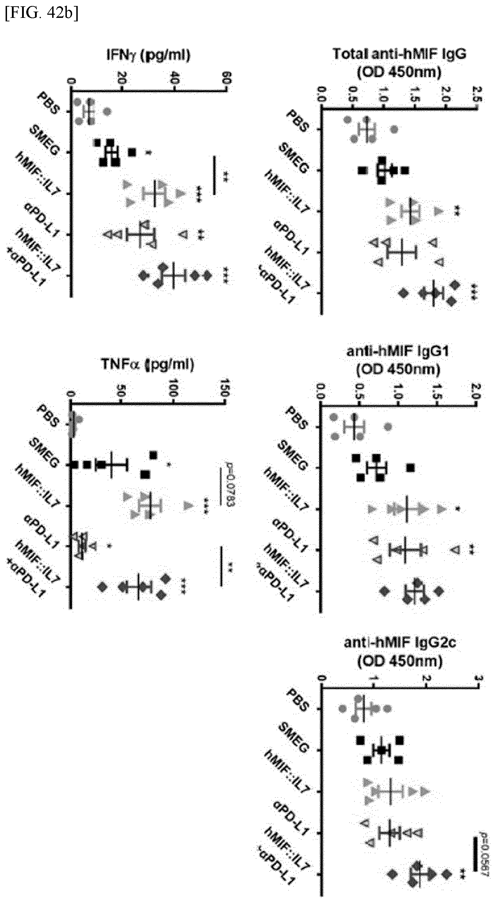

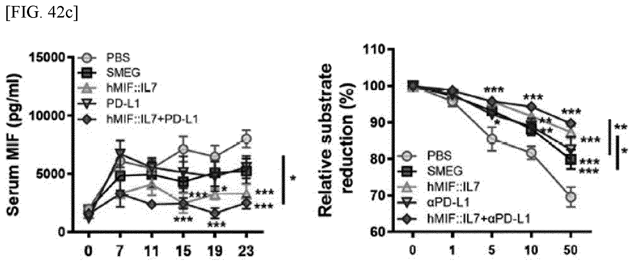

In addition, it was confirmed that, when *M. smegmatis*-pMyong2_hMIF::IL7 was administered alone, the humoral immune response against MIF in serum increased, and this immune response more effectively increased when administered when co-administered anti-PD-L1. Moreover, it was confirmed that the concentrations of inflammatory cytokines in serum effectively increased when *M. smegmatis-*pMyong2_hMIF::IL7 and anti-PD-L1 were co-administered compared to when they were administered alone, and that the concentration of MIF in serum was the least when they were co-administered (FIG. 42b). Therefore, it could be seen that the concentration of MIF in serum is the lowest when *M. smegmatis-*pMyong2_hMIF::IL7 and anti-PD-L1 were co-administered, and the biological activity of serum MIF, that is, the tautomerase activity, was also the lowest when they were co-administered (FIG. 42c).

These results suggest that the previously observed effects of administration of *M. smegmatis*-pMyong2_hMIF::IL7 alone on cancer growth inhibition and the immune response against MIF in serum are maximized when *M. smegmatis*-pMyong2_hMIF::IL7 is co-administered with anti-PD-L1, which is a commercially available anticancer drug.

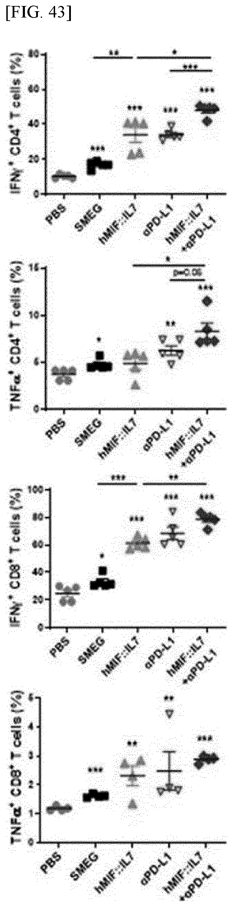

Next, in order to examine the degree of activation of the cellular immune response in tumor tissue upon co-administration of *M. smegmatis*-pMyong2_hMIF::IL7 and anti-PD-L1, tumor tissue was isolated and T cells secreting cytokines were measured through flow cytometry. In the same manner as the previous experiment, when *M. smegmatis*-pMyong2_hMIF::IL7 was administered alone, the proportions of CD4 helper T cells and cytotoxic CD8 T cells secreting cytokines IFNy and TNFα with anti-cancer effects among immune cells that infiltrated into tumor tissue increased, and this effect was further increased when anti-PD-L1 and *M. smegmatis*-pMyong2_hMIF::IL7 were co-administered (FIG. 43a).

Taking the above experimental results together, it could be seen that the increase in the immune response against MIF and the increase in immune cells secreting cytokines in the tumor, which were observed when *M. smegmatis*-pMyong2_hMIF::IL7 was administered alone, were further effectively increased when *M. smegmatis-*pMyong2_hMIF::IL7 was co-administered with anti-PD-L1, which is a currently commercially available anticancer drug.

### Evaluation of cytotoxicity against MC38 by immune cells infected with recombinant strain

Naive CD8 T cells isolated from the spleen using BD Aria were co-cultured with dendritic cells infected with *M. smegmatis*-pMyong2_hMIF::IL7 for 4 days (dendritic cells: T cells = 1:10). Thereafter, MC38 cancer cells were co-cultured with T cells at a ratio of 1:5 for 2 days. The cytotoxic ability of the immune cells infected with the recombinant strain against cancer cells was evaluated using 7AAD/Annexin V apoptosis assay, and the cytokine expression levels in the immune cells at the same time point were compared.

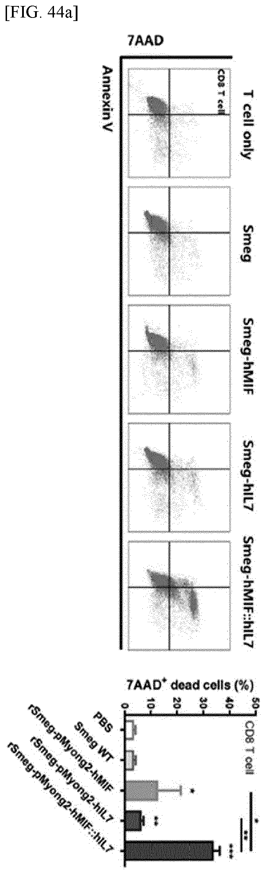

It was confirmed that, when the *M. smegmatis*-pMyong2_hMIF::IL7-infected dendritic cells were co-cultured with the CD8 T cells, the proportion of dead MC38 cancer cells was most increased in the case of the *M. smegmatis*-pMyong2_hMIF::IL7 strain compared to the other recombinant strains, suggesting that *M. smegmatis-*pMyong2_hMIF::IL7 induced cytotoxicity against the cancer cells more effectively than the recombinant strains introduced with each of the hMIF and hIL7 proteins (FIG. 44a).

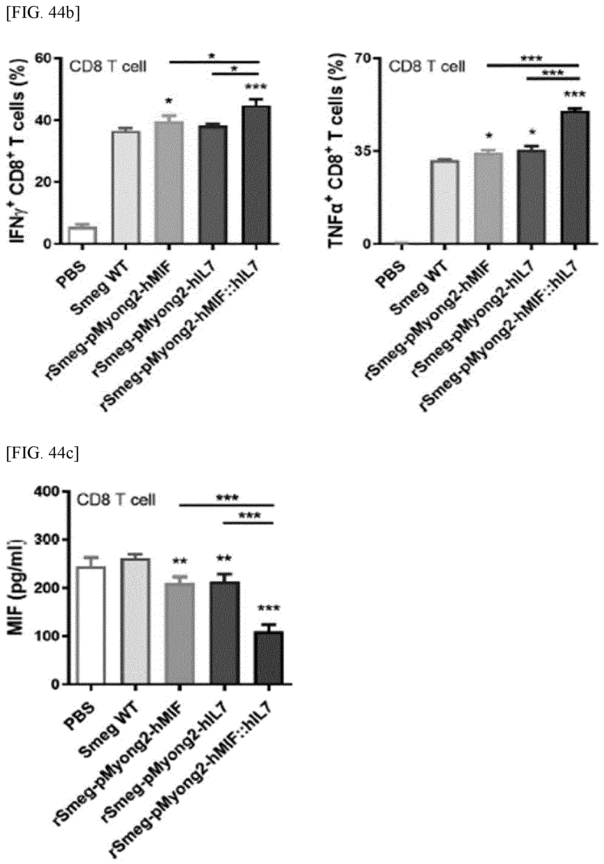

In addition, the cytotoxicity against cancer cells induced by *M. smegmatis-*pMyong2_hMIF::IL7 was validated through cytokine expression in immune cells. That is, it could be seen that, when the *M. smegmatis-*pMyong2_hMIF::IL7-infected immune cells were co-cultured with cancer cells, the proportion of immune cells secreting IFNy and TNFα having anti-cancer effects was higher than when immune cells infected with the other recombinant strains were co-cultured with cancer cells, and thus the most cancer cells were killed (FIG. 44b).

As a result of analyzing the concentration of MIF in the co-culture of the infected immune cells and the cancer cells through ELISA, it was confirmed that the concentration of MIF significantly decreased when the immune cells were infected with *M. smegmatis-*pMyong2_hMIF::IL7 compared to when they were infected with the other recombinant strains (FIG. 44c). This is believed to be because the proportion of killed cancer cells increased due to M. smegmatis-pMyong2_hMIF::IL7 infection, resulting in a decrease in the amount of MIF derived from the cancer cells (FIG. 44c).

### Observation of anticancer effect against various cancer cell lines

In order to evaluate *in vitro* the anticancer effect of the recombinant strains obtained in the above Example, various human and mouse cancer cells (human liver cancer cells Huh7 and HepG2, mouse pancreatic cancer cells PanO2, mouse lung epithelial cells TC-1, mouse lung cancer cells LLC, and mouse melanoma cells B16F10) were infected with each recombinant strain of the present invention, and then the metabolic activity of the cancer cells was measured by the MTS cell proliferation assay kit (Promega, USA), thereby evaluating the toxicity of the recombinant strain against the cancer cells.

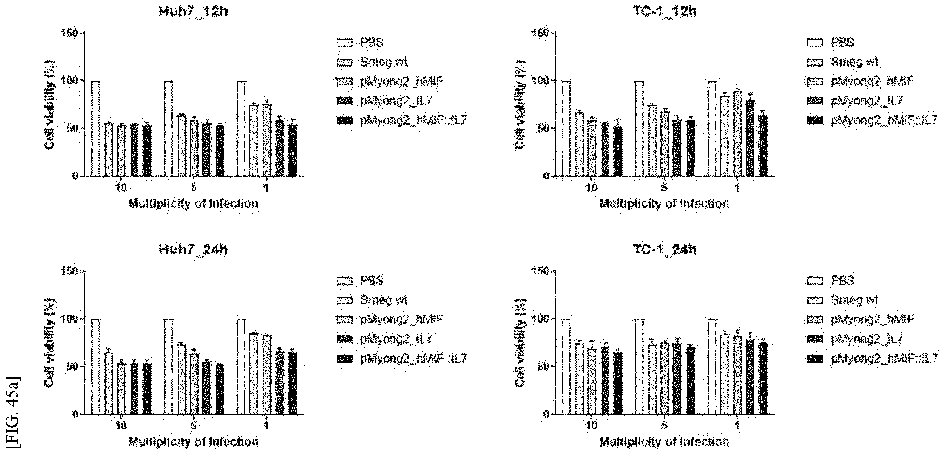

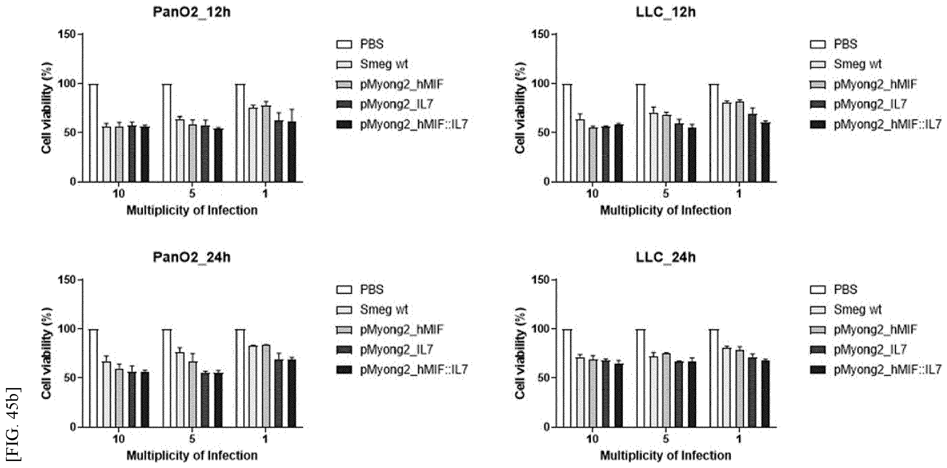

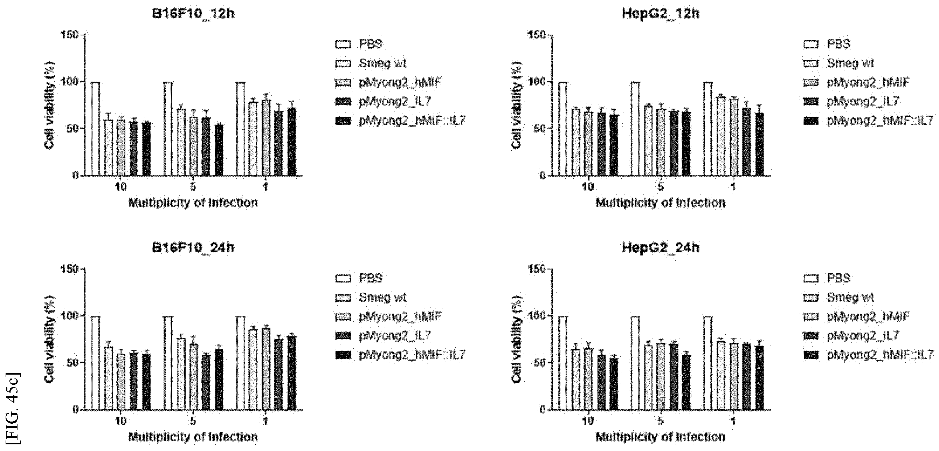

As a result, it was confirmed that, in all of the Huh7, HepG2, TC-1, PanO2, LLC, and B16F10 cell lines, the recombinant strains transformed with the pMyong2 vector had better cytotoxicity against the cancer cells than the recombinant strain transformed with the pMV306 vector. (FIG. 45). This property is thought to be due to the significantly higher expression levels of human MIF and human IL-7 in the pMyong2-TOPO vector than in the pMV306 vector, as confirmed in the performance of the recombinant strain observed above. It was confirmed again using the various cancer cell lines that a remarkable synergistic effect was exhibited when human MIF and human IL-7 were expressed as a fusion protein compared to each of the proteins was expressed alone.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A mycobacteria-derived replicable plasmid comprising: a nucleic acid molecule encoding macrophage migration inhibitory factor (MIF) or a functional portion thereof; a nucleic acid molecule encoding interleukin-7 (IL-7) or a functional portion thereof; or a combination thereof.

2. The plasmid of claim 1, further comprising an origin of replication comprising the nucleotide sequence of SEQ ID NO: 2 and a promoter of hsp65 or hsp60 gene.

3. The plasmid of claim 2, which is a pMyong2 plasmid shown in FIG. 1a.

4. The plasmid of claim 3, comprising the nucleotide sequence of SEQ ID NO: 1.

5. A recombinant *Mycobacterium* strain comprising the plasmid of any one of claims 1 to 4.

6. The strain of claim 5, wherein the *Mycobacterium* is selected from the group consisting of *M. smegmatis*, *M. bovis* BCG, *M. avium*, *M. phlei*, *M. fortuitum*, *M. lufu*, *M. partuberculosis*, *M. habana*, *M. scrofulaceum*, and *M. intracellulare.*

7. The strain of claim 6, wherein the *Mycobacterium* is *M. smegmatis.*

8. A composition for preventing or treating cancer comprising the strain of claim 5 as an active ingredient.

9. The composition of claim 8, wherein the cancer is metastatic cancer.

10. The composition of claim 8, further comprising cisplatin or a pharmaceutically acceptable salt thereof

11. The composition of claim 8, further comprising an immune checkpoint inhibitor.

12. The composition of claim 11, wherein the immune checkpoint inhibitor is anti-PD-L1 antibody.
